# EUROPEAN PATENT SPECIFICATION

(11) **EP 1 458 395 B1**
(45) Date of publication and mention of the grant of the patent: **28.11.2007**
(21) Application number: 02793677.2
(22) Date of filing: 18.12.2002
(51) Int. Cl.: A61K 31/517, A61P 25/28, A61P 25/14, A61P 25/18, A61P 25/24, A61P 9/10, A61P 15/16

(54) **MEDICAL USE OF GSK3-INHIBITING OXINDOLE DERIVATIVES**
MEDIZINISCHE VERWENDUNG VON GSK3-HEMMENDEN OXINDOLDERIVATEN
UTILISATION DE DERIVES OXINDOLIQUES INHIBANT LA GSK3

(30) Priority: 20.12.2001 SE 0104341
(43) Date of publication of application: 22.09.2004
(73) Proprietor: AstraZeneca AB, 151 85 Södertälje (SE)
(72) Inventor: BERG, Stefan, S-151 85 Södertälje (SE); BHAT, Ratan, S-151 85 Södertälje (SE); HELLBERG, Sven, S-151 85 Södertälje (SE)
(86) International application number: PCT/SE2002/002372
(87) International publication number: WO 2003/053444

(56) References cited:
- EP-A1- 1 136 493
- WO-A1-00/10975
- WO-A1-95/33750
- WO-A1-97/42187
- WO-A1-99/10349
- HEWAWASAM PIYASENA ET AL.: 'Synthesis and structure-activity relationships of 3-aryloxindoles: A new class of calcium-dependent, large conductance potassium (Maxi-K) channel openers with neuroprotective properties' J. MED. CHEM. vol. 45, 2002, pages 1487 - 1499, XP002959071
- IMAHORI AND UCHIDA: J. BIOCHEM, vol. 121, 1997, pages 179-188,
- HOSHI ET AL.: PNAS, vol. 93, 1996, pages 2719-2723,
- BHAT ET AL. PNAS: PNAS, vol. 97, 2000, pages 11074-11079,
- STAMBOLIC ET AL. 6: CURR. BIOL., vol. 6, 1996, pages 1664-1668,
- COTTER ET AL. 9: 1379-1383: NEUROREPORT, vol. 9, 1998, pages 1379-1383,
- KOZLOVSKY ET AL.: AM J PSYCHIATRY, vol. 157, 2000, pages 831-833,
- GAT ET AL.: CELL, vol. 95, 1998, pages 605-614,

## Description

### FIELD OF INVENTION

The present invention relates to a new use of specific oxindole derivatives, as a free base or salts thereof, in the manufacture of a medicament for the treatment and/or prevention of Alzheimer's Disease, Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia, dementia pugilistica, amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, Mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment.

### BACKGROUND OF THE INVENTION

Glycogen synthase kinase 3 (GSK3) is a serine / threonine protein kinase composed of two isoforms (α and β), which are encoded by distinct genes but are highly homologous within the catalytic domain. GSK3 is highly expressed in the central and peripheral nervous system. GSK3 phosphorylates several substrates including tau, β-catenin, glycogen synthase, pyruvate dehydrogenase and elongation initiation factor 2b (eIF2b). Insulin and growth factors activate protein kinase B, which phosphorylates GSK3 on the serine 9 residue and inactivates it.

### Alzheimer's Disease (AD) dementias, and taupathies.

AD is characterized by cognitive decline, cholinergic dysfunction and neuronal death, neurofibrillary tangles and senile plaques consisting of amyloid-β deposits. The sequence of these events in AD is unclear, but believed to be related. Glycogen synthase kinase 3β (GSK3β) or Tau (τ) phosphorylating kinase selectively phosphorylates the microtubule associated protein τ in neurons at sites that are hyperphosphorylated in AD brains. Hyperphosphorylated protein τ has lower affinity for microtubules and accumulates as paired helical filaments, which are the main components that constitute neurofibrillary tangles and neuropil threads in AD brains. This results in depolymerization of microtubules, which leads to dying back of axons and neuritic dystrophy. Neurofibrillary tangles are consistently found in diseases such as AD, amyotrophic lateral sclerosis, parkinsonism-dementia complex of Gaum, corticobasal degeneration, dementia pugilistica and head trauma, Down's syndrome, postencephalatic parkinsonism, progressive supranuclear palsy, Niemann-Pick's Disease and Pick's Disease. Addition of amyloid-β to primary hippocampal cultures results in hyperphosphorylation of τ and a paired helical filaments-like state via induction of GSK3β activity, followed by disruption of axonal transport and neuronal death (Imahori and Uchida., J. Biochem 121:179-188, 1997). GSK3β preferentially labels neurofibrillary tangles and has been shown to be active in pre-tangle neurons in AD brains. GSK3 protein levels are also increased by 50% in brain tissue from AD patients. Furthermore, GSK3β phosphorylates pyruvate dehydrogenase, a key enzyme in the glycolytic pathway and prevents the conversion of pyruvate to acetyl-Co-A (Hoshi et al., PNAS 93:2719-2723, 1996). Acetyl-Co-A is critical for the synthesis of acetylcholine, a neurotransmitter with cognitive functions. Thus, GSK3β inhibition may have beneficial effects in progression as well as the cognitive deficits associated with Alzheimer's disease and other above-referred to diseases.

### Chronic and Acute Neurodegenerative Diseases.

Growth factor mediated activation of the PI3K /Akt pathway has been shown to play a key role in neuronal survival. The activation of this pathway results in GSK3β inhibition. Recent studies (Bhat et. al., PNAS 97:11074-11079 (2000)) indicate that GSK3β activity is increased in cellular and animal models of neurodegeneration such as cerebral ischemia or after growth factor deprivation. For example, the active site phosphorylation was increased in neurons vulnerable to apoptosis, a type of cell death commonly thought to occur in chronic and acute degenerative diseases such as Alzheimer's Disease, Parkinson's Disease, amyotrophic lateral sclerosis, Huntington's Disease and HIV dementia, ischemic stroke and head trauma. Lithium was neuroprotective in inhibiting apoptosis in cells and in the brain at doses that resulted in the inhibition of GSK3β. Thus GSK3β inhibitors could be useful in attenuating the course of neurodegenerative diseases.

### Bipolar Disorders (BD)

Bipolar Disorders are characterised by manic episodes and depressive episodes. Lithium has been used to treat BD based on its mood stabilising effects. The disadvantage of lithium is the narrow therapeutic window and the danger of overdosing that can lead to lithium intoxication. The recent discovery that lithium inhibits GSK3 at therapeutic concentrations has raised the possibility that this enzyme represents a key target of lithium's action in the brain (Stambolic et al., Curr. Biol. 6:1664-1668, 1996; Klein and Melton; PNAS 93:8455-8459, 1996). Inhibition of GSK3β may therefore be of therapeutic relevance in the treatment of BD as well as in AD patients that have affective disorders.

### Schizophrenia

GSK3 is involved in signal transduction cascades of multiple cellular processes, particularly during neural development. Kozlovsky et al (Am J Psychiatry 2000 May;157(5):831-3) found that GSK3β levels were 41% lower in the schizophrenic patients than in comparison subjects. This study indicates that schizophrenia involves neurodevelopmental pathology and that abnormal GSK3 regulation could play a role in schizophrenia. Furthermore, reduced β-catenin levels have been reported in patients exhibiting schizophrenia (Cotter et al., Neuroreport 9:1379-1383 (1998)).

### DETAILED DESCRIPTION OF THE INVENTION

Compounds of general formula I are disclosed in WO 99/10349. The effect of the compounds on reducing antiangiogenic and/or vascular permeability in mammals has been investigated.

It has now suprisingly been found that the group of oxindole derivatives as decribed in WO 99/10349 are well suited for inhibiting glycogen synthase kinase-3. Said glycogen synthase kinase-3 inhibitors are suitable in the prevention and/or treatment of conditions associated with glycogen synthase kinase-3 in the central and peripheral nervous system. In particular, the compounds of the invention are expected to be suitable for prevention and/or treatment of Alzheimer's Disease, Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica.

The compounds of the invention are also expected to be suitable for prevention and/or treatment of amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases Bipolar Disease, affective disorders, depression, schizophrenia, and cognitive disorders.

The compounds of the invention are further expected to be suitable for prevention and/or treatment of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment.

In the present invention GSK3 inhibitors of general formula I may be used in the manufacturing of a medicament for the treatment and/or prevention of Alzheimer's Disease, Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia, dementia pugilistica, amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss, contraceptive medication, Mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment and androgenetic alopecia. 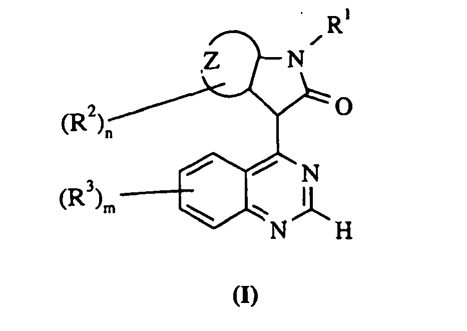 wherein:
ring Z is a 5 or 6 membered heterocyclic ring containing 1 to 3 heteroatoms selected independently from O, N and S but not more than 2 nitrogen atom;
R¹ is hydrogen or C₁₋₃alkyl;
R² is hydroxy, halogeno, fluoromethyl, difluoromethyl, trifluoromethyl, fluoromethoxy, difluoromethoxy, trifluoromethoxy, 2,2,2-trifluoroethyl, cyano, amino, nitro, C₁₋₃alkyl, C₁₋₃alkoxy, C₁₋₃alkanoyloxy, C₂₋₄alkanoyl, C₁₋₄alkanoylamino, C₁₋₄alkoxycarbonyl, C₁₋₄alkylthio, C₁₋₄alkylsulphinyl, C₁₋₄alkylsulphonyl, carbamoyl,*N*-C₁₋₄alkylcarbamoyl, *N,N*-di(C₁₋₄alkyl)carbamoyl, aminosulphonyl, *N*-C₁₋₄alkylaminosulphonyl, *N,N*-di(C₁₋₄alkyl)aminosulphonyl or C₁₋₄alkylsulphonylamino, or
R² is selected from one of the following groups:
   1) R⁴X¹, wherein X¹ is a direct bond, O, NR⁵*,* C₁₋₃alkyl, C₂₋₄alkanoyl, CONR⁶R⁷, SO₂NR⁸R⁹ or SO₂R¹⁰ (wherein R⁵, R⁶ and R⁸ each independently represent hydrogen or C₁₋₂alkyl and R⁷, R⁹ and R¹⁰ each independently represent C₁₋₄alkyl and wherein
      R⁴ is linked to R⁷, R⁹ or R¹⁰); and
      R⁴ is phenyl or a 5 or 6 membered heterocyclic group with one or two heteroatoms, selected independently from O, S and N, which heterocyclic group may be saturated or unsaturated and which phenyl or heterocyclic group may be substituted with one or two substituents selected independently from oxo, hydroxy, halogeno, C₁₋₃alkyl, C₁₋₃alkoxy, C₁₋₃alkanoyloxy, trifluoromethyl, cyano, amino, nitro and C₁₋₄alkoxycarbonyl;
   2) X²C₂₋₄alkylX³C₁₋₃alkyl (wherein X² is O or NR¹¹ (wherein R¹¹ is hydrogen, C₁₋₃alkyl or C₁₋₃alkoxyC₂₋₃alkyl) and X³ is O, NR¹², S, SO or SO₂ (wherein R¹² is hydrogen, C₁₋₃alkyl or C₁₋₃alkoxyC₂₋₃alkyl));
   3) C₁₋₂alkylX⁴C₂₋₃alkylX⁵C₁₋₃alkyl (wherein X⁴ and X⁵ each independently represent O, S, SO, SO₂ or NR¹³ (wherein R¹³ is hydrogen, C₁₋₃alkyl or C₁₋₃alkoxyC₂₋₃alkyl)); and
   4) C₁₋₃alkylX⁶C₁₋₃alkyl (wherein X⁶ is O, S, SO, SO₂ or NR¹⁴ (wherein R¹⁴ is hydrogen, C₁₋₃alkyl or C₁₋₃alkoxyC₂₋₃alkyl));
R³ is hydroxy, halogeno, nitro, fluoromethyl, difluoromethyl, trifluoromethyl, fluoromethoxy, difluoromethoxy, trifluoromethoxy, C₁₋₃alkyl, cyano, amino or R¹⁵X⁷,
wherein X⁷ is a direct bond, O, CH₂, S, SO, SO₂, NR¹⁶CO, CONR¹⁷, SO₂NR¹⁸, NR¹⁹SO₂ or NR²⁰ (wherein R¹⁶,R¹⁷, R¹⁸, R¹⁹ and R²⁰ each independently represent hydrogen, C₁₋₃alkyl or C₁₋₃alkoxyC₂₋₃alkyl); and
R¹⁵ is selected from one of the following groups:
   1) hydrogen or C₁₋₅alkyl, which may be substituted with one or more groups selected independently from hydroxy, fluoro and amino;
   2) C₁₋₅alkylX⁸COR²¹ (wherein X⁸ is O or NR²² (wherein R²² is hydrogen, C₁₋₃alkyl or C₁₋₃alkoxyC₂₋₃alkyl) and R²¹ is C₁₋₃alkyl, NR²³R²⁴ or OR²⁵ (wherein R²³, R²⁴ and R²⁵ each independently represent hydrogen, C₁₋₃alkyl or C₁₋₃alkoxyC₂₋₃alkyl));
   3) C₁₋₅alkylX⁹R²⁶ (wherein X⁹ is O, S, SO, SO₂, OCO, NR²⁷CO, CONR²⁸, SO₂NR²⁹, NR³⁰SO₂ or NR³¹ (wherein R²⁷, R²⁸, R²⁹, R³⁰ and R³¹ each independently represent hydrogen, C₁₋₃alkyl or C₁₋₃alkoxyC₂₋₃alkyl) and R²⁶ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl,
      C₁₋₄hydroxyalkyl and C₁₋₄alkoxy);
   4) C₁₋₅alkylX¹⁰C₁₋₅alkylX¹¹R³² (wherein X¹⁰ and X¹¹ each independently represent O, S, SO,SO₂, NR³³CO, CONR³⁴, SO₂NR³⁵, NR³⁶SO₂ or NR³⁷ (wherein R³³, R³⁴, R³⁵, R³⁶ and R³⁷ each independently represent hydrogen, C₁₋₃alkyl or C₁₋₃alkoxyC₂₋₃alkyl) and R³² is hydrogen or C₁₋₃alkyl);
   5) R³⁸ (wherein R³⁸ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
   6) C₁₋₅alkylR³⁸ (wherein R³⁸ is as defined hereinbefore);
   7) C₂₋₅alkenylR³⁸ (wherein R³⁸ is as defined hereinbefore);
   8) C₂₋₅alkynylR³⁸ (wherein R³⁸ is as defined hereinbefore);
   9) R³⁹ (wherein R³⁹ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, trifluoromethyl, cyano, CONR⁴⁰R⁴¹ and NR⁴²COR⁴³ (wherein R⁴⁰, R⁴¹, R⁴² and R⁴³ each independently represent hydrogen, C₁₋₄alkyl or C₁₋₃alkoxyC₂₋₃alkyl));
   10) C₁₋₅alkylR³⁹ (wherein R³⁹ is as defined hereinbefore);
   11) C₂₋₅alkenylR³⁹ (wherein R³⁹ is as defined hereinbefore);
   12) C₂₋₅alkynylR³⁹ (wherein R³⁹ is as defined hereinbefore);
   13) C₁₋₅alkylX¹²R³⁹ (wherein X¹² is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently represent hydrogen, C₁₋₃alkyl or C₁₋₃alkoxyC₂₋₃alkyl) and R³⁹ is as defined hereinbefore);
   14) C₂₋₅alkenylX¹³R³⁹ (wherein X¹³ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently represent hydrogen, C₁₋₃alkyl or C₁₋₃alkoxyC₂₋₃alkyl) and R³⁹ is as defined hereinbefore);
   15) C₂₋₅alkynylX¹⁴R³⁹ (wherein X¹⁴ is O, S, SO, SO₂, NR⁵⁴CO, CONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently represent hydrogen, C₁₋₃alkyl or C₁₋₃alkoxyC₂₋₃alkyl) and R³⁹ is as defined hereinbefore);
   16) C₁₋₃alkylX¹⁵C₁₋₃alkylR³⁹ (wherein X¹⁵ is O, S, SO, SO₂, NR⁵⁹CO, CONR⁶⁰, SO₂NR⁶¹, NR⁶²SO₂ or NR⁶³ (wherein R⁵⁹, R⁶⁰, R⁶¹, R⁶² and R⁶³ each independently represent hydrogen, C₁₋₃alkyl or C₁₋₃alkoxyC₂₋₃alkyl) and R³⁹ is as defined hereinbefore); and
   17) C₁₋₃alkylX¹⁵C₁₋₃alkylR³⁸ (wherein X¹⁵ and R³⁸ are as defined hereinbefore);
n is 0, 1, 2 or 3 when Z is a 6 membered heterocyclic ring and n is 0, 1 or 2 when Z is a 5 membered heterocyclic ring;
m is 0, 1, 2, 3 or 4;
as a free base or pharmaceutically acceptable salts thereof.

According to one aspect of the present invention compounds of formula I may be used,
wherein R¹, R², R³, m and n are as defined hereinbefore; and
ring Z is a 6 membered heterocyclic ring containing 1 or 2 nitrogen atoms.

In another aspect of the invention compounds of formula I may be used, wherein Z is a 6 membered heterocyclic ring containing 1 or 2 nitrogen atoms and R¹ is hydrogen.

In a further aspect of the invention compounds of formula I may be used, wherein R² is halogeno, C₁₋₃alkyl, trifluoromethyl, cyano, carbamoyl, *N*-C₁₋₄alkylcarbamoyl, aminosulphonyl or a group R⁴X¹,
wherein X¹ is CONR⁶R⁷ (wherein R⁶ is hydrogen or C₁₋₂alkyl and R⁷ is C₁₋₄alkyl and
wherein R⁴ is linked to R⁷); and
n is 0 or 1.

In yet another aspect of the invention compounds of formula I may be used, wherein R³ is R¹⁵X⁷,
wherein X⁷ is O; and
R¹⁵ is selected from one of the following groups:
1) hydrogen or C₁₋₅alkyl;
3) C₁₋₅alkylX⁹R²⁶ (wherein X⁹ is O (wherein R²⁶ is hydrogen or C₁₋₃alkyl));
4) C₁₋₅alkylX¹⁰C₁₋₅alkylX¹¹R³² (wherein X¹⁰ and X¹¹ are O, and R³² is hydrogen or C₁₋₃alkyl);
6) C₁₋₅alkylR³⁸ (wherein R³⁸ is a 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
7) C₂₋₅alkenylR³⁸ (wherein R³⁸ is as defined hereinbefore);
10) C₁₋₅alkylR³⁹ (wherein R³⁹ is a 5 or 6 membered aromatic heterocyclic group with 1 to 3 heteroatoms selected independently from O, N and S, which heterocyclic group may carry up to 4 substituents selected independently from hydroxy halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, trifluoromethyl, cyano, CONR⁴⁰R⁴¹ and NR⁴²COR⁴³ (wherein R⁴⁰, R⁴¹, R⁴² and R⁴³ each independently represent hydrogen, C₁₋₄alkyl or C₁₋₃alkoxyC₂₋₃alkyl));
13) C₁₋₅alkylX¹²R³⁹ (wherein X¹² is O and R³⁹ is as defined hereinbefore);
m is 0, 1 or 2.

One aspect of the invention relates to the use of compounds of formula I, wherein R¹ is hydrogen, Z is a 6 membered heterocyclic ring containing 1 or 2 nitrogen atoms, R² is halogeno or C₁₋₃alkyl and n is 0 or 1, R³ is morpholinopropoxy, dioxothiomorpholino-propoxy, morpholinobutenyl-oxy, pyridyloxy-ethoxy, triazolyl-ethoxy, imidazolyl-ethoxy, methoxy, methoxyethoxy or methoxyethoxy-ethoxy and m is 0, 1, or 2.

In another aspect of the invention, use is made of the following compounds in the manufacturing of a medicament for the treatment and/or prevention of conditions associated with glycogen synthase kinase-3;
4-(7-Azaoxindol-3-yl)-6-methoxy-7-(2-methoxyethoxy)quinazoline,
4-(7-Azaoxindol-3-yl)-6-methoxy-7-(3-morpholinopropoxy)quinazoline,
4-(7-Azaoxindol-3-yl)-7-(2-(imidazol-1-yl)ethoxy)-6-methoxyquinazoline,
4-(5,7-Diaza-6-methyloxindol-3-yl)-7-(3-morpholinopropoxy)quinazoline,
4-(7-Aza-6-chlorooxindol-3-yl)-7-(3-morpholinopropoxy)quinazoline,
4-(5,7-Diaza-6-methyloxindol-3-yl)-6-methoxy-7- (2-(1,2,3-triazol-1-yl)ethoxy)quinazoline,
4-(5,7-Diaza-6-methyloxindol-3-yl)-7-(2-(2-methoxyethoxy)ethoxy)quinazoline,
4-(7-Azaoxindol-3-yl)-7-(3-morpholinopropoxy)quinazoline,
4-(7-Azaoxindol-3-yl)-7-(2-(2-methoxyethoxy)ethoxy)quinazoline,
4-(7-Azaoxindol-3-yl)-7-(4-morpholinobut-2-en-1-yloxy)quinazoline,
4-(5,7-Diaza-6-methyloxindol-3-yl)-6-methoxy-7-(2-(4-pyridyloxy)ethoxy)quinazoline,
4-(7-Aza-6-chlorooxindol-3-yl)-7-(2-(2-methoxyethoxy)ethoxy)quinazoline, and
4-(5,7-Diaza-6-methyloxindol-3-yl)-7-(3-(1,1-dioxothiomorpholino)-propoxy)quinazoline;
as a free base or pharmaceutically acceptable salts thereof.

For the avoidance of doubt it is to be understood that where in this specification a group is qualified by 'hereinbefore defined' or 'defined hereinbefore' the said group encompasses the first occurring and broadest definition as well as each and all of the preferred definitions of that group.

For the avoidance of doubt it is to be understood that in this specification 'C₁₋₅' means a carbon group having 1, 2, 3, 4 or 5 carbon atoms.

In this specification, unless stated otherwise, the term "alkyl" includes both straight and branched chain alkyl groups. C₁₋₅alkyl may be methyl, ethyl, n-propyl, i-propyl, n-butyl, i-butyl, s-butyl, t-butyl, n-pentyl, i-pentyl, t-pentyl, neo-pentyl.

The term "alkoxy" as used herein, unless stated otherwise includes "alkyl"O groups in which "alkyl" is as hereinbefore defined. C₁₋₅alkoxy may be methoxy, ethoxy, n-propoxy, i-propoxy, n-butoxy, i-butoxy, s-butoxy, t-butoxy, n-pentyloxy, i-pentyloxy, t-pentyloxy, neo-pentyloxy.

The term "alkanoyl" as used herein, unless otherwise stated includes formyl and alkylC=O groups in which "alkyl" is as defined hereinbefore, for example C₂alkanoyl is ethanoyl and refers to CH₃C=O, C₁alkanoyl is formyl and refers to CHO.

In this specification, unless stated otherwise, the term "alkenyl" includes both straight and branched chain alkenyl groups but references to individual alkenyl groups such as 2-butenyl are specific for the straight chain version only. Unless otherwise stated, the term "alkenyl" advantageously refers to chains with 2 to 5 carbon atoms, preferably 3 to 4 carbon atoms.

In this specification, unless stated otherwise, the term "alkynyl" includes both straight and branched chain alkynyl groups but references to individual alkynyl groups such as 2-butynyl are specific for the straight chain version only. Unless otherwise stated, the term "alkynyl" advantageously refers to chains with 2 to carbon atoms, preferably 3 to 4 carbon atoms.

In this specification, unless stated otherwise, the term "5 or 6 membered heterocyclic group with one or two heteroatoms, selected independently from O, S and N, which heterocyclic group may be saturated or unsaturated" or "5 or 6 membered heterocyclic ring containing 1 to 3 heteroatoms selected independently from O, N and S, which heterocyclic group may be saturated or unsaturated", includes both heteroaromatic rings and heterocyclic rings that are saturated. Examples of such heterocyclic groups includes furyl, imidazolyl, isoxazolyl, isothiazolyl, oxazolyl, pyrazinyl, pyrazolyl, pyridazinyl, pyridyl, pyrimidyl, pyrrolyl, thiazolyl, thienyl, imidazolidinyl, imidazolinyl, morpholinyl, piperazinyl, piperidyl, piperidonyl, pyrazolidinyl, pyrazolinyl, pyrrolidinyl, pyrrolinyl, tetrahydropyranyl or thiomorpholinyl.

In this specification, unless stated otherwise, the term "5 or 6 membered saturated heterocyclic group with one or two heteroatoms, selected independently from O, S and N" may be imidazolidinyl, imidazolinyl, morpholinyl, piperazinyl, piperidyl, piperidonyl, pyrazolidinyl, pyrazolinyl, pyrrolidinyl, pyrrolinyl, tetrahydropyranyl or thiomorpholinyl. In this specification, unless stated otherwise, the term "5 or 6 membered aromatic heterocyclic group with 1 to 3 heteroatoms, selected independently from O, N and S" may be furyl, imidazolyl, isoxazolyl, isothiazolyl, oxazolyl, pyrazinyl, triazinyl, pyrazolyl, pyridazinyl, pyridyl, pyrimidyl, pyrrolyl, thiazolyl or thienyl.

In this specification, unless stated otherwise, the term "5 or 6 membered heterocyclic ring containing 1 to 3 heteroatoms selected independently from O, N and S" may be furyl, imidazolyl, isoxazolyl, isothiazolyl, oxazolyl, pyrazinyl, pyrazolyl, pyridazinyl, pyridyl, pyrimidyl, pyrrolyl, thiazolyl or thienyl.

In this specification, unless stated otherwise, the term halogeno may be fluor, chlorine, bromine or iodine.

For the avoidance of any doubt, it is to be understood that when X⁷ is, for example, a group of formula NR¹⁶CO, it is the nitrogen atom be substituted withing the R¹⁶ group which is attached to the quinazoline ring and the carbonyl (CO) group is attached to R¹⁵, whereas when X⁷ is, for example, a group of formula CONR¹⁷, it is the carbonyl group which is attached to the quinazoline ring and the nitrogen atom be substituted withing the R¹⁷ group is attached to R¹⁵. A similar convention applies to the other two atoms X⁷ linking groups such as NR¹⁹SO₂ and SO₂NR¹⁸. When X⁷ is NR²⁰ it is the nitrogen atom be substituted withing the R²⁰ group, which is linked to the quinazoline ring and to R¹⁵. An analogous convention applies to other groups. It is further to be understood that when X⁷ represents NR²⁰ and R²⁰ is C₁₋₃alkoxyC₂₋₃alkyl it is the C₂₋₃alkyl moiety, which is linked to the nitrogen atom of X⁷ and an analogous convention applies to other groups.

For the avoidance of any doubt, it is to be understood that in a compound of formula I when R¹⁵ is, for example, a group of formula C₁₋₅alkylX¹⁵C₁₋₅alkylR³⁹, it is the terminal C₁₋₅alkyl moiety, which is linked to X¹⁵, similarly when R¹⁵ is, for example, a group of formula C₂₋₅alkenylR³⁹ it is the C₂₋₅alkenyl moiety, which is linked to X⁷ and an analogous convention applies to other groups.

For the avoidance of any doubt, it is to be understood that when R³⁹ carries a C₁₋₄aminoalkyl substituent it is the C₁₋₄alkyl moiety, which is attached to R³⁹ whereas when R³⁹ carries a C₁₋₄alkylamino substituent it is the amino moiety, which is attached to R³⁹ and an analogous convention applies to other groups.

For the avoidance of any doubt when X¹ is C₂₋₄alkanoyl it is the carbonyl moiety, which is linked to the heteroaromatic oxindole group and it is the alkyl moiety, which is linked to R⁴ and an analogous convention applies to other groups.

For the avoidance of any doubt when R² is a group X²C₂₋₄alkylX³C₁₋₃alkyl it is X², which is linked to the heteroaromatic oxindole group and an analogous convention applies to other groups. When R² is a group C₁₋₂alkylX⁴C₂₋₃alkylX⁵C₁₋₃alkyl it is the C₁₋₂alkyl moiety, which is linked to the heteroaromatic oxindole group and an analogous convention applies to other groups.

Some compounds of formula I may have chiral centres and/or geometric isomeric centres (E- and Z- isomers), and it is to be understood that the invention encompasses all such optical, diastereoisomers and geometric isomers that possess GSK3 inhibitory activity.

It is to be understood that the present invention also relates to any and all tautomeric forms of the compounds of formula **I**.

The present invention relates to the use of compounds of formula **I** as hereinbefore defined as well as to the salts thereof. Salts for use in pharmaceutical compositions will be pharmaceutically acceptable salts, but other salts may be useful in the production of the compounds of formula **I.**

Both organic and inorganic acids can be employed to form non-toxic pharmaceutically acceptable acid addition salts of the compounds of this invention. In addition, a suitable pharmaceutically acceptable salt of the compounds of the invention is an alkali metal salt, an alkaline earth metal salt or a salt with an organic base.

Compound of formula I, or salt thereof, may be prepared by any process known to be applicable to the preparation of chemically-related compounds. Such processes include, for example, those illustrated in European Patent Applications Publication Nos. 0520722, 0566226, 0602851, 0635498 and 0636608 and PCT application WO 99/10349.

### Pharmaceutical composition

The composition may be in a form suitable for oral administration, for example as a tablet, pill, syrup, powder, granule or capsule, for parenteral injection (including intravenous, subcutaneous, intramuscular, intravascular or infusion) as a sterile solution, suspension or emulsion, for topical administration as an ointment, patch or cream or for rectal administration as a suppository.

In general the above compositions may be prepared in a conventional manner using pharmaceutically acceptable carriers or diluents.

Suitable daily doses of the compounds of formula I in the treatment of a mammal, including man, are approximately 0.01 to 250 mg/kg bodyweight at peroral administration and about 0.001 to 250 mg/kg bodyweight at parenteral administration. The typical daily dose of the active ingredients varies within a wide range and will depend on various factors such as the relevant indication, the route of administration, the age, weight and sex of the patient and may be determined by a physician.

Illustrate representative pharmaceutical dosage forms containing a compound of formula I, as a free base or salts thereof, are described in WO 99/10349.

### Medical use

The compounds of the invention are expected to be suitable in the manufacture of a medicament for the prevention and/or treatment of Alzheimer's Disease, Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica. The compounds of the invention are also expected to be suitable in the manufacture of a medicament for the prevention and/or treatment of amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia and, cognitive disorders.

The compounds of the invention are further expected to be suitable in the manufacture of a medicament for the prevention and/or treatment of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment.

In the context of the present specification, the term "therapy" also includes "prevention" unless there are specific indications to the contrary. The terms "therapeutic" and "therapeutically" should be construed accordingly.

### Pharmacology

### Determination of ATP competition in Scintillation Proximity GSK3βAssay.

### GSK3β scintillation proximity assay.

The competition experiments were carried out in duplicate with 10 different concentrations of the inhibitors in clear-bottom microtiter plates (Wallac, Finland). A biotinylated peptide substrate, Biotin-Ala-Ala-Glu-Glu-Leu-Asp-Ser-Arg-Ala-Gly-Ser(PO₃H₂)-Pro-Gln-Leu (AstraZeneca, Lund), was added at a final concentration of 1 µM in an assay buffer containing 1 mU recombinant human GSK3β (Dundee University, UK), 12 mM morpholinepropanesulfonic acid (MOPS), pH 7.0, 0.3 mM EDTA, 0.01 % β-mercaptorethanol, 0.004 % Brij 35 (a natural detergent), 0.5 % glycerol and 0.5 µg BSA/25 µl. The reaction was initiated by the addition of 0.04 µCi [γ-³³P]ATP (Amersham, UK) and unlabelled ATP at a final concentration of 1 µM and assay volume of 25 µl. After incubation for 20 minutes at room temperature, each reaction was terminated by the addition of 25 µl stop solution containing 5 mM EDTA, 50 µM ATP, 0.1 % Triton X-100 and 0.25 mg streptavidin coated Scintillation Proximity Assay (SPA) beads (Amersham, UK). After 6 hours the radioactivity was determined in a liquid scintillation counter (1450 MicroBeta Trilux, Wallac). The inhibition curves were analysed by non-linear regression using GraphPad Prism, USA. The Kₘ value of ATP for GSK3β, used to calculate the inhibition constants (Kᵢ) of the various compounds, was 20 µM.

The following abbreviations have been used:
- ATP: Adenosine Triphophatase
- BSA: Bovin Serum Albumin
- EDTA: Ethylenediaminetetraacetic acid
- GSK3: Glycogen synthase kinase 3
- MOPS: Morpholinepropanesulfonic acid
- SPA: Scintillation Proximity Assay

### Results

Typical Kᵢ values for the compounds of the present invention are in the range of about 0.001 to about 10,000 nM. Other values for Kᵢ are in the range of about 0.001 to about 1000 nM. Further values for Kᵢ are in the range of about 0.001 nM to about 300 nM.

## Claims

1. Use of a compound of formula I 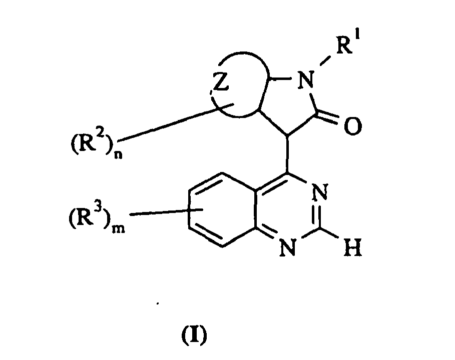 wherein:
ring Z is a 5 or 6 membered heterocyclic ring containing 1 to 3 heteroatoms selected independently from O, N and S but not more than 2 nitrogen atoms;
R¹ is hydrogen or C₁₋₃alkyl;
R² is hydroxy, halogeno, fluoromethyl, difluoromethyl, trifluoromethyl, fluoromethoxy, difluoromethoxy, trifluoromethoxy, 2,2,2-trifluoroethyl, cyano, amino, nitro, C₁₋₃alkyl, C₁₋₃alkoxy, C₁₋₃alkanoyloxy, C₂₋₄alkanoyl, C₁₋₄alkanoylamino, C₁₋₄alkoxycarbonyl, C₁₋₄alkylthio, C₁₋₄alkylsulphinyl, C₁₋₄alkylsulphonyl, carbamoyl, *N*-C₁₋₄alkylcarbamoyl, *N,N-*di(C₁₋₄alkyl)carbamoyl, aminosulphonyl, *N*-C₁₋₄alkylaminosulphonyl, *N,N*-di(C₁₋₄alkyl)aminosulphonyl or C₁₋₄alkylsulphonylamino, or
R² is selected from one of the following groups:
1) R⁴X¹, wherein X¹ is a direct bond, O, NR⁵, C₁₋₃alkyl, C₂₋₄alkanoyl, CONR⁶R⁷, SO₂NR⁸R⁹ or SO₂R¹⁰ (wherein R⁵, R⁶ and R⁸ each independently represent hydrogen or C₁₋₂alkyl and R⁷, R⁹ and R¹⁰ each independently represent C₁₋₄alkyl and wherein R⁴ is linked to R⁷, R⁹ or R¹⁰); and
R⁴ is phenyl or a 5 or 6 membered heterocyclic group with one or two heteroatoms, selected independently from O, S and N, which heterocyclic group may be saturated or unsaturated and which phenyl or heterocyclic group may be substituted with one or two substituents selected independently from oxo, hydroxy, halogeno, C₁₋₃alkyl, C₁₋₃alkoxy, C₁₋₃alkanoyloxy, trifluoromethyl, cyano, amino, nitro and C₁₋₄alkoxycarbonyl;
2) X²C₂₋₄alkylX³C₁₋₃alkyl (wherein X² is O or NR¹¹ (wherein R¹¹ is hydrogen, C₁₋₃alkyl or C₁₋₃alkoxyC₂₋₃alkyl) and X³ is O, NR¹², S, SO or SO₂ (wherein R¹² is hydrogen, C₁₋₃alkyl or C₁₋₃alkoxyC₂₋₃alkyl));
3) C₁₋₂alkylX⁴C₂₋₃alkylX⁵C₁₋₃alkyl (wherein X⁴ and X⁵ each independently represent O, S, SO, SO₂ or NR¹³ (wherein R¹³ is hydrogen, C₁₋₃alkyl or C₁₋₃alkoxyC₂₋₃alkyl)); and
4) C₁₋₃alkylX⁶C₁₋₃alkyl (wherein X⁶ is O, S, SO, SO₂ or NR¹⁴ (wherein R¹⁴ is hydrogen, C₁₋₃alkyl or C₁₋₃alkoxyC₂₋₃alkyl));
R³ is hydroxy, halogeno, nitro, fluoromethyl, difluoromethyl, trifluoromethyl, fluoromethoxy, difluoromethoxy, trifluoromethoxy, C₁₋₃alkyl, cyano, amino or R¹⁵X⁷,
wherein X⁷ is a direct bond, O, CH₂, S, SO, SO₂, NR¹⁶CO, CONR¹⁷, SO₂NR¹⁸, NR¹⁹SO₂ or NR²⁰ (wherein R¹⁶, R¹⁷, R¹⁸, R¹⁹ and R²⁰ each independently represent hydrogen, C₁₋₃alkyl or C₁₋₃alkoxyC₂₋₃alkyl); and
R¹⁵ is selected from one of the following groups:
1) hydrogen or C₁₋₅alkyl, which may be substituted with one or more groups selected independently from hydroxy, fluoro and amino;
2) C₁₋₅alkylX⁸COR²¹ (wherein X⁸ is O or NR²² (wherein R²² is hydrogen, C₁₋₃alkyl or C₁₋₃alkoxyC₂₋₃alkyl) and R²¹ is C₁₋₃alkyl, NR²³R²⁴ or OR²⁵ (wherein R²³, R²⁴ and R²⁵ each independently represent hydrogen, C₁₋₃alkyl or C₁₋₃alkoxyC₂₋₃alkyl));
3) C₁₋₅alkylX⁹R²⁶ (wherein X⁹ is O, S, SO, SO₂, OCO, NR²⁷CO, CONR²⁸, SO₂NR²⁹, NR³⁰SO₂ or NR³¹ (wherein R²⁷, R²⁸, R²⁹, R³⁰ and R³¹ each independently represent hydrogen, C₁₋₃alkyl or C₁₋₃alkoxyC₂₋₃alkyl) and R²⁶ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl,
C₁₋₄hydroxyalkyl and C₁₋₄alkoxy);
4) C₁₋₅alkylX¹⁰C₁₋₅alkylX¹¹R³² (wherein X¹⁰ and X¹¹ each independently represent O, S, SO, SO₂, NR³³CO, CONR³⁴, SO₂NR³⁵, NR³⁶SO₂ or NR³⁷ (wherein R³³, R³⁴, R³⁵, _{R}36 and R³⁷ each independently represent hydrogen, C₁₋₃alkyl or C₁₋₃alkoxyC₂₋₃alkyl) and R³² is hydrogen or C₁₋₃alkyl);
5) R³⁸ (wherein R³⁸ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
6) C₁₋₅alkylR³⁸ (wherein R³⁸ is as defined hereinbefore);
7) C₂₋₅alkenylR³⁸ (wherein R³⁸ is as defined hereinbefore);
8) C₂₋₅alkynylR³⁸ (wherein R³⁸ is as defined hereinbefore);
9) R³⁹ (wherein R³⁹ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, trifluoromethyl, cyano, CONR⁴⁰R⁴¹ and NR⁴²COR⁴³ (wherein R⁴⁰, R⁴¹, R⁴² and R⁴³ each independently represent hydrogen, C₁₋₄alkyl or C₁₋₃alkoxyC₂₋₃alkyl));
10) C₁₋₅alkylR³⁹ (wherein R³⁹ is as defined hereinbefore);
11) C₂₋₅alkenylR³⁹ (wherein R³⁹ is as defined hereinbefore);
12) C₂₋₅alkynylR³⁹ (wherein R³⁹ is as defined hereinbefore);
13) C₁₋₅alkylX¹²R³⁹ (wherein X¹² is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently represent hydrogen, C₁₋₃alkyl or C₁₋₃alkoxyC₂₋₃alkyl) and R³⁹ is as defined hereinbefore);
14) C₂₋₅alkenylX¹³R³⁹ (wherein X¹³ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently represent hydrogen, C₁₋₃alkyl or C₁₋₃alkoxyC₂₋₃alkyl) and R³⁹ is as defined hereinbefore);
15) C₂₋₅alkynylX¹⁴R³⁹ (wherein X¹⁴ is O, S, SO, SO₂, NR⁵⁴CO, CONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently represent hydrogen, C₁₋₃alkyl or C₁₋₃alkoxyC₂₋₃alkyl) and R³⁹ is as defined hereinbefore);
16) C₁₋₃alkylX¹⁵C₁₋₃alkylR³⁹ (wherein X¹⁵ is O, S, SO, SO₂, NR⁵⁹CO, CONR⁶⁰, SO₂NR⁶¹, NR⁶²SO₂ or NR⁶³ (wherein R⁵⁹, R⁶⁰, R⁶¹, R⁶² and R⁶³ each independently represent hydrogen, C₁₋₃alkyl or C₁₋₃alkoxyC₂₋₃alkyl) and R³⁹ is as defined hereinbefore); and
17) C₁₋₃alkylX¹⁵C₁₋₃alkylR³⁸ (wherein X¹⁵ and R³⁸ are as defined hereinbefore);
n is 0, 1, 2 or 3 when Z is a 6 membered heterocyclic ring and n is 0, 1 or 2 when Z is a 5 membered heterocyclic ring;
m is 0, 1, 2, 3 or 4;
as a free base or pharmaceutically acceptable salts thereof, in the manufacturing of a medicament for the treatment and/or prevention of
Alzheimer's Disease, Frontotemporal dementia Parkinson's Type, Parkinson dementia complex of Guam, HIV dementia, predemented states, vascular dementia, dementia with Lewy bodies, Frontotemporal dementia and dementia pugilistica.

2. Use of a compound of formula I 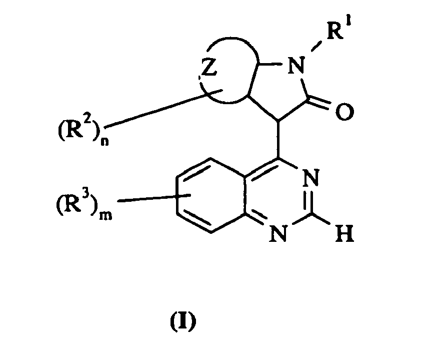 wherein:
ring Z is a 5 or 6 membered heterocyclic ring containing 1 to 3 heteroatoms selected independently from O, N and S but not more than 2 nitrogen atom;
R¹ is hydrogen or C₁₋₃alkyl;
R² is hydroxy, halogeno, fluoromethyl, difluoromethyl, trifluoromethyl, fluoromethoxy, difluoromethoxy, trifluoromethoxy, 2,2,2-trifluoroethyl, cyano, amino, nitro, C₁₋₃alkyl, C₁₋₃alkoxy, C₁₋₃alkanoyloxy, C₂₋₄alkanoyl, C₁₋₄alkanoylamino, C₁₋₄alkoxycarbonyl, C₁₋₄alkylthio, C₁₋₄alkylsulphinyl, C₁₋₄alkylsulphonyl, carbamoyl,*N*-C₁₋₄alkylcarbamoyl, *N,N*-di(C₁₋₄alkyl)carbamoyl, aminosulphonyl, *N*-C₁₋₄alkylaminosulphonyl, *N,N*-di(C₁₋₄alkyl)aminosulphonyl or C₁₋₄alkylsulphonylamino, or
R² is selected from one of the following groups:
1) R⁴X¹, wherein X¹ is a direct bond, O, NR⁵, C₁₋₃alkyl, C₂₋₄alkanoyl, CONR⁶R⁷, SO₂NR⁸R⁹ or SO₂R¹⁰ (wherein R⁵, R⁶ and R⁸ each independently represent hydrogen or C₁₋₂alkyl and R⁷, R⁹ and R¹⁰ each independently represent C₁₋₄alkyl and wherein R⁴ is linked to R⁷, R⁹ or R¹⁰); and
R⁴ is phenyl or a 5 or 6 membered heterocyclic group with one or two heteroatoms, selected independently from O, S and N, which heterocyclic group may be saturated or unsaturated and which phenyl or heterocyclic group may be substituted with one or two substituents selected independently from oxo, hydroxy, halogeno, C₁₋₃alkyl, C₁₋₃alkoxy, C₁₋₃alkanoyloxy, trifluoromethyl, cyano, amino, nitro and
C₁₋₄alkoxycarbonyl;
2) X²C₂₋₄alkylX³C₁₋₃alkyl (wherein X² is O or NR¹¹ (wherein R¹¹ is hydrogen, C₁₋₃alkyl or C₁₋₃alkoxyC₂₋₃alkyl) and X³ is O, NR¹², S, SO or SO₂ (wherein R¹² is hydrogen, C₁₋₃alkyl or C₁₋₃alkoxyC₂₋₃alkyl));
3) C₁₋₂alkylX⁴C₂₋₃alkylX⁵C₁₋₃alkyl (wherein X⁴ and X⁵ each independently represent O, S, SO, SO₂ or NR¹³ (wherein R¹³ is hydrogen, C₁₋₃alkyl or C₁₋₃alkoxyC₂₋₃alkyl)); and
4) C₁₋₃alkylX⁶C₁₋₃alkyl (wherein X⁶ is O, S, SO, SO₂ or NR¹⁴ (wherein R¹⁴ is hydrogen, C₁₋₃alkyl or C₁₋₃alkoxyC₂₋₃alkyl));
R³ is hydroxy, halogeno, nitro, fluoromethyl, difluoromethyl, trifluoromethyl, fluoromethoxy, difluoromethoxy, trifluoromethoxy, C₁₋₃alkyl, cyano, amino or R¹⁵X⁷,
wherein X⁷ is a direct bond, O, CH₂, S, SO, SO₂, NR¹⁶CO, CONR¹⁷, SO₂NR¹⁸, NR¹⁹SO₂ or NR²⁰ (wherein R¹⁶, R¹⁷, R¹⁸, R¹⁹ and R²⁰ each independently represent hydrogen, C₁₋₃alkyl or C₁₋₃alkoxyC₂₋₃alkyl); and
R¹⁵ is selected from one of the following groups:
1) hydrogen or C₁₋₅alkyl, which may be substituted with one or more groups selected independently from hydroxy, fluoro and amino;
2) C₁₋₅alkylX⁸COR²¹ (wherein X⁸ is O or NR²² (wherein R²² is hydrogen, C₁₋₃alkyl or C₁₋₃alkoxyC₂₋₃alkyl) and R²¹ is C₁₋₃alkyl, NR²³R²⁴ or OR²⁵ (wherein R²³, R²⁴ and R²⁵ each independently represent hydrogen, C₁₋₃alkyl or C₁₋₃alkoxyC₂₋₃alkyl));
3) C₁₋₅alkylX⁹R²⁶ (wherein X⁹ is O, S, SO, SO₂, OCO, NR²⁷CO, CONR²⁸, SO₂NR²⁹, NR³⁰SO₂ or NR³¹ (wherein R²⁷, R²⁸, R²⁹, R³⁰ and R³¹ each independently represent hydrogen, C₁₋₃alkyl or C₁₋₃alkoxyC₂₋₃alkyl) and R²⁶ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl,
C₁₋₄hydroxyalkyl and C₁₋₄alkoxy);
4) C₁₋₅alkylX¹⁰C₁₋₅alkylX¹¹R³² (wherein X¹⁰ and X¹¹ each independently represent O, S, SO, SO₂, NR³³CO, CONR³⁴, SO₂NR³⁵, NR³⁶SO₂ or NR³⁷ (wherein R³³,R³⁴, R³⁵, R³⁶ and R³⁷ each independently represent hydrogen, C₁₋₃alkyl or C₁₋₃alkoxyC₂₋₃alkyl) and R³² is hydrogen or C₁₋₃alkyl);
5) R³⁸ (wherein R³⁸ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
6) C₁₋₅alkylR³⁸ (wherein R³⁸ is as defined hereinbefore);
7) C₂₋₅alkenylR³⁸ (wherein R³⁸ is as defined hereinbefore);
8) C₂₋₅alkynylR³⁸ (wherein R³⁸ is as defined hereinbefore);
9) R³⁹ (wherein R³⁹ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, trifluoromethyl, cyano, CONR⁴⁰R⁴¹ and NR⁴²COR⁴³ (wherein R⁴⁰, R⁴¹, R⁴² and R⁴³ each independently represent hydrogen, C₁₋₄alkyl or C₁₋₃alkoxyC₂₋₃alkyl));
10) C₁₋₅alkylR³⁹ (wherein R³⁹ is as defined hereinbefore);
11) C₂₋₅alkenylR³⁹ (wherein R³⁹ is as defined hereinbefore);
12) C₂₋₅alkynylR³⁹ (wherein R³⁹ is as defined hereinbefore);
13) C₁₋₅alkyl¹²R³⁹ (wherein X¹² is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently represent hydrogen, C₁₋₃alkyl or C₁₋₃alkoxyC₂₋₃alkyl) and R³⁹ is as defined hereinbefore);
14) C₂₋₅alkenylX¹³R³⁹ (wherein X¹³ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently represent hydrogen, C₁₋₃alkyl or C₁₋₃alkoxyC₂₋₃alkyl) and R³⁹ is as defined hereinbefore);
15) C₂₋₅alkynylX¹⁴R³⁹ (wherein X¹⁴ is O, S, SO, SO₂, NR⁵⁴CO, CONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently represent hydrogen, C₁₋₃alkyl or C₁₋₃alkoxyC₂₋₃alkyl) and R³⁹ is as defined hereinbefore);
16) C₁₋₃alkylX¹⁵C₁₋₃alkylR³⁹ (wherein X¹⁵ is O, S, SO, SO₂, NR⁵⁹CO, CONR⁶⁰, SO₂NR⁶¹, NR⁶²SO₂ or NR⁶³(wherein R⁵⁹, R⁶⁰, R⁶¹, R⁶² and R⁶³ each independently represent hydrogen, C₁₋₃alkyl or C₁₋₃alkoxyC₂₋₃alkyl) and R³⁹ is as defined hereinbefore); and
17) C₁₋₃alkylX¹⁵C₁₋₃alkylR³⁸ (wherein X¹⁵ and R³⁸ are as defined hereinbefore);
n is 0, 1, 2 or 3 when Z is a 6 membered heterocyclic ring and n is 0, 1 or 2 when Z is a 5 membered heterocyclic ring;
m is 0, 1, 2, 3 or 4;
as a free base or pharmaceutically acceptable salts thereof, in the manufacturing of a medicament for the treatment and/or prevention of
amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, and cognitive disorders.

3. Use of a compound of formula **I** 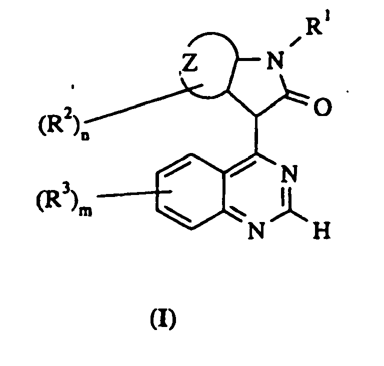 wherein:
ring Z is a 5 or 6 membered heterocyclic ring containing 1 to 3 heteroatoms selected independently from O, N and S but not more than 2 nitrogen atom;
R¹ is hydrogen or C₁₋₃alkyl;
R² is hydroxy, halogeno, fluoromethyl, difluoromethyl, trifluoromethyl, fluoromethoxy, difluoromethoxy, trifluoromethoxy, 2,2,2-trifluoroethyl, cyano, amino, nitro, C₁₋₃alkyl, C₁₋₃alkoxy, C₁₋₃alkanoyloxy, C₂₋₄alkanoyl, C₁₋₄alkanoylamino, C₁₋₄alkoxycarbonyl, C₁₋₄alkylthio, C₁₋₄alkylsulphinyl, C₁₋₄alkylsulphonyl, carbamoyl,*N*-C₁₋₄alkylcarbamoyl, *N,N*-di(C₁₋₄alkyl)carbamoyl, aminosulphonyl, *N*-C₁₋₄alkylaminosulphonyl, *N,N*-di(C₁₋₄alkyl)aminosulphonyl or C₁₋₄alkylsulphonylamino, or
R² is selected from one of the following groups:
1) R⁴X¹, wherein X¹ is a direct bond, O, NR⁵, C₁₋₃alkyl, C₂₋₄alkanoyl, CONR⁶R⁷, SO₂NR⁸R⁹ or SO₂R¹⁰ (wherein R⁵, R⁶ and R⁸ each independently represent hydrogen or C₁₋₂alkyl and R⁷, R⁹ and R¹⁰ each independently represent C₁₋₄alkyl and wherein R⁴ is linked to R⁷, R⁹ or R¹⁰); and
R⁴ is phenyl or a 5 or 6 membered heterocyclic group with one or two heteroatoms, selected independently from O, S and N, which heterocyclic group may be saturated or unsaturated and which phenyl or heterocyclic group may be substituted with one or two substituents selected independently from oxo, hydroxy, halogeno, C₁₋₃alkyl, C₁₋₃alkoxy, C₁₋₃alkanoyloxy, trifluoromethyl, cyano, amino, nitro and
C₁₋₄alkoxycarbonyl;
2) X²C₂₋₄alkylX³C₁₋₃alkyl (wherein X² is O or NR¹¹ (wherein R¹¹ is hydrogen, C₁₋₃alkyl or C₁₋₃alkoxyC₂₋₃alkyl) and X³ is O, NR¹², S, SO or SO₂ (wherein R¹² is hydrogen, C₁₋₃alkyl or C₁₋₃alkoxyC₂₋₃alkyl));
3) C₁₋₂alkylX⁴C₂₋₃alkylX⁵C₁₋₃alkyl (wherein X⁴ and X⁵ each independently represent O, S, SO, SO₂ or NR¹³ (wherein R¹³ is hydrogen, C₁₋₃alkyl or C₁₋₃alkoxyC₂₋₃alkyl)); and
4) C₁₋₃alkyX⁶C₁₋₃alkyl (wherein X⁶ is O, S, SO, SO₂ or NR¹⁴ (wherein R¹⁴ is hydrogen, C₁₋₃alkyl or C₁₋₃alkoxyC₂₋₃alkyl));
R³ is hydroxy, halogeno, nitro, fluoromethyl, difluoromethyl, trifluoromethyl, fluoromethoxy, difluoromethoxy, trifluoromethoxy, C₁₋₃alkyl, cyano, amino or R¹⁵X⁷,
wherein X⁷ is a direct bond, O, CH₂, S, SO, SO₂, NR¹⁶CO, CONR¹⁷, SO₂NR¹⁸, NR¹⁹SO₂ or NR²⁰ (wherein R¹⁶, R¹⁷, R¹⁸, R¹⁹ and R²⁰ each independently represent hydrogen, C₁₋₃alkyl or C₁₋₃alkoxyC₂₋₃alkyl); and
R¹⁵ is selected from one of the following groups:
1) hydrogen or C₁₋₅alkyl, which may be substituted with one or more groups selected independently from hydroxy, fluoro and amino;
2) C₁₋₅alkylX⁸COR²¹ (wherein X⁸ is O or NR²² (wherein R²² is hydrogen, C₁₋₃alkyl or C₁₋₃alkoxyC₂₋₃alkyl) and R²¹ is C₁₋₃alkyl, NR²³R²⁴ or OR²⁵ (wherein R²³, R²⁴ and R²⁵ each independently represent hydrogen, C₁₋₃alkyl or C₁₋₃alkoxyC₂₋₃alkyl));
3) C₁₋₅alkylX⁹R²⁶ (wherein X⁹ is O, S, SO, SO₂, OCO, NR²⁷CO, CONR²⁸, SO₂NR²⁹, NR³⁰SO₂ or NR³¹ (wherein R²⁷, R²⁸, R²⁹, R³⁰ and R³¹ each independently represent hydrogen, C₁₋₃alkyl or C₁₋₃alkoxyC₂₋₃alkyl) and R²⁶ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl,
C₁₋₄hydroxyalkyl and C₁₋₄alkoxy);
4) C₁₋₅alkylX¹⁰C₁₋₅alkylX¹¹R³² (wherein X¹⁰ and X¹¹ each independently represent O, S, SO, SO₂, NR³³CO, CONR³⁴, SO₂NR³⁵, NR³⁶SO₂ or NR³⁷ (wherein R³³, R³⁴, R³⁵, R³⁶ and R³⁷ each independently represent hydrogen, C₁₋₃alkyl or C₁₋₃alkoxyC₂₋₃alkyl) and R³² is hydrogen or C₁₋₃alkyl);
5) R³⁸ (wherein R³⁸ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
6) C₁₋₅alkylR³⁸ (wherein R³⁸ is as defined hereinbefore);
7) C₂₋₅alkenylR³⁸ (wherein R³⁸ is as defined hereinbefore);
8) C₂₋₅alkynylR³⁸ (wherein R³⁸ is as defined hereinbefore);
9) R³⁹ (wherein R³⁹ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, trifluoromethyl, cyano, CONR⁴⁰R⁴¹ and NR⁴²COR⁴³ (wherein R⁴⁰, R⁴¹, R⁴² and R⁴³ each independently represent hydrogen, C₁₋₄alkyl or C₁₋₃alkoxyC₂₋₃alkyl));
10) C₁₋₅alkylR³⁹ (wherein R³⁹ is as defined hereinbefore);
11) C₂₋₅alkenylR³⁹ (wherein R³⁹ is as defined hereinbefore);
12) C₂₋₅alkynylR³⁹ (wherein R³⁹ is as defined hereinbefore);
13) C₁₋₅alkylX¹²R³⁹ (wherein X¹² is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently represent hydrogen, C₁₋₃alkyl or C₁₋₃alkoxyC₂₋₃alkyl) and R³⁹ is as defined hereinbefore);
14) C₂₋₅alkenylX¹³R³⁹ (wherein X¹³ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently represent hydrogen, C₁₋₃alkyl or C₁₋₃alkoxyC₂₋₃alkyl) and R³⁹ is as defined hereinbefore);
15) C₂₋₅alkynylX¹⁴R³⁹ (wherein X¹⁴ is O, S, SO, SO₂, NR⁵⁴CO, CONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently represent hydrogen, C₁₋₃alkyl or C₁₋₃alkoxyC₂₋₃alkyl) and R³⁹ is as defined hereinbefore);
16) C₁₋₃alkylX¹⁵C₁₋₃alkylR³⁹ (wherein X¹⁵ is O, S, SO, SO₂, NR⁵⁹CO, CONR⁶⁰, SO₂NR⁶¹, NR⁶²SO₂ or NR⁶³ (wherein R⁵⁹, R⁶⁰, R⁶¹, R⁶² and R⁶³ each independently represent hydrogen, C₁₋₃alkyl or C₁₋₃alkoxyC₂₋₃alkyl) and R³⁹ is as defined hereinbefore); and
17) C₁₋₃alkylX¹⁵C₁₋₃alkylR³⁸ (wherein X¹⁵ and R³⁸ are as defined hereinbefore);
n is 0, 1, 2 or 3 when Z is a 6 membered heterocyclic ring and n is 0, 1 or 2 when Z is a 5 membered heterocyclic ring;
m is 0, 1, 2, 3 or 4;
as a free base or pharmaceutically acceptable salts thereof, in the manufacturing of a medicament for the treatment and/or prevention of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment.

4. The use according to any one of claims 1-3, wherein Z is a 6 membered heterocyclic ring containing 1 or 2 nitrogen atoms and R¹ is hydrogen.

5. The use according to any one of claims 1-4, wherein R² is halogeno, C₁-₃alkyl, trifluoromethyl, cyano, carbamoyl, *N*-C₁₋₄alkylcarbamoyl, aminosulphonyl or a group R⁴X¹,
wherein X¹ is CONR⁶R⁷ (wherein R⁶ is hydrogen or C₁₋₂alkyl and R⁷ is C₁₋₄alkyl and
wherein R⁴ is linked to R⁷); and
n is 0 or 1.

6. The use according to any one of claims 1-5,
wherein R³ is R¹⁵X⁷,
wherein X⁷ is O; and
R¹⁵ is selected from one of the following groups:
1) hydrogen or C₁₋₅alkyl;
3) C₁₋₅alkylX⁹R²⁶ (wherein X⁹ is O (wherein R²⁶ is hydrogen or C₁₋₃alkyl));
4) C₁₋₅alkylX¹⁰C₁₋₅alkylX¹¹R³² (wherein X¹⁰ and X¹¹ are O, and R³² is hydrogen or C₁₋₃alkyl);
6) C₁₋₅alkylR³⁸ (wherein R³⁸ is a 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
7) C₂₋₅alkenylR³⁸ (wherein R³⁸ is as defined hereinbefore);
10) C₁₋₅alkylR³⁹ (wherein R³⁹ is a 5 or 6 membered aromatic heterocyclic group with 1 to 3 heteroatoms selected independently from O, N and S, which heterocyclic group may carry up to 4 substituents selected independently from hydroxy halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, trifluoromethyl, cyano, CONR⁴⁰R⁴¹ and NR⁴²COR⁴³ (wherein R⁴⁰, R⁴¹, R⁴² and R⁴³ each independently represent hydrogen, C₁₋₄alkyl or C₁₋₃alkoxyC₂₋₃alkyl));
13) C₁₋₅alkylX¹²R³⁹ (wherein X¹² is O and R³⁹ is as defined hereinbefore);
m is 0, 1 or 2.

7. The use according to any one of claims 1-6, wherein the compounds are selected from
4-(7-Azaoxindol-3-yl)-6-methoxy-7-(2-methoxyethoxy)quinazoline,
4-(7-Azaoxindol-3-yl)-6-methoxy-7-(3-morpholinopropoxy)quinazoline,
4-(7-Azaoxindol-3-yl)-7-(2-(imidazol-1-yl)ethoxy)-6-methoxyquinazoline,
4-(5,7-Diaza-6-methyloxindol-3-yl)-7-(3-morpholinopropoxy)quinazoline,
4-(7-Aza-6-chlorooxindol-3-yl)-7-(3-morpholinopropoxy)quinazoline,
4-(5,7-Diaza-6-methyloxindol-3-yl)-6-methoxy-7-(2-(1,2,3-triazol-1-yl)ethoxy)quinazoline,
4-(5,7-Diaza-6-methyloxindol-3-yl)-7-(2-(2-methoxyethoxy)ethoxy)quinazoline,
4-(7-Azaoxindol-3-yl)-7-(3-morpholinopropoxy)quinazoline,
4-(7-Azaoxindol-3-yl)-7-(2-(2-methoxyethoxy)ethoxy)quinazoline,
4-(7-Azaoxindol-3-yl)-7-(4-morpholinobut-2-en-1-yloxy)quinazoline,
4-(5,7-Diaza-6-methyloxindol-3-yl)-6-methoxy-7-(2-(4-pyridyloxy)ethoxy)quinazoline,
4-(7-Aza-6-chlorooxindol-3-yl)-7-(2-(2-methoxyethoxy)ethoxy)quinazoline, and
4-(5,7-Diaza-6-methyloxindol-3-yl)-7-(3-(1,1-dioxothiomorpholino)-propoxy)quinazoline;
as a free base or pharmaceutically acceptable salts thereof.

## Patentansprüche

1. Verwendung einer Verbindung der Formel I 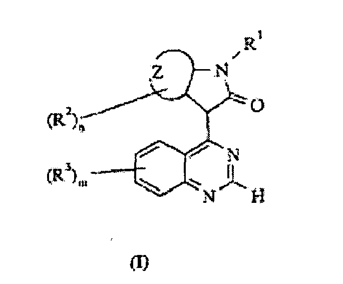 wobei:
Ring Z für einen 5- oder 6-gliedrigen heterocyclischen Ring mit 1 bis 3 Heteroatomen unabhängig voneinander ausgewählt aus O, N und S, jedoch nicht mit mehr als 2 Stickstoffatomen, steht;
R¹ für Wasserstoff oder C₁₋₃-Alkyl steht;
R² für Hydroxy, Halogen, Fluormethyl, Difluormethyl, Trifluormethyl, Fluormethoxy, Difluormethoxy, Trifluormethoxy, 2,2,2-Trifluorethyl, Cyano, Amino, Nitro, C₁₋₃-Alkyl, C₁₋₃-Alkoxy, C₁₋₃-Alkanoyloxy, C₂₋₄-Alkanoyl, C₁₋₄-Alkanoylamino, C₁₋₄-Alkoxycarbonyl, C₁₋₄-Alkylthio, C₁₋₄-Alkylsulfinyl, C₁₋₄-Alkylsulfonyl, Carbamoyl, *N*-C₁₋₄-Alkylcarbamoyl, *N*,*N*-Di (C₁₋₄-alkyl)carbamoyl, Aminosulfonyl, *N*-C₁₋₄-Alkylaminosulfonyl, *N,N-*Di(C₁₋₄-alkyl)aminosulfonyl oder C₁₋₄-Alkylsulfonylamino steht, oder
R² ausgewählt ist aus einer der folgenden Gruppen:
1) R⁴X¹, wobei X¹ für eine direkte Bindung, 0, NR⁵, C₁₋₃-Alkyl, C₂₋₄-Alkanoyl, CONR⁶R⁷, SO₂NR⁸R⁹ oder SO₂R¹⁰ steht (wobei R⁵, R⁶ und R⁸ jeweils unabhängig voneinander für Wasserstoff oder C₁₋₂-Alkyl stehen und R⁷, R⁹ und R¹⁰ jeweils unabhängig voneinander für C₁₋₄-Alkyl stehen und wobei R⁴ an R⁷, R⁹ oder R¹⁰ gebunden ist); und
R⁴ für Phenyl oder eine 5- oder 6-gliedrige heterocyclische Gruppe mit einem oder zwei Heteroatomen unabhängig voneinander ausgewählt aus O, S und N steht, wobei die heterocyclische Gruppe gesättigt oder ungesättigt sein kann und wobei die Phenylgruppe bzw. die heterocyclische Gruppe durch einen oder zwei Substituenten unabhängig voneinander ausgewählt aus Oxo, Hydroxy, Halogen, C₁₋₃-Alkyl, C₁₋₃-Alkoxy, C₁₋₃-Alkanoyloxy, Trifluormethyl, Cyano, Amino, Nitro und C₁₋₄-Alkoxycarbonyl substituiert sein kann;
2) X²-C₂₋₄-Alkyl-X³-C₁₋₃-alkyl (wobei X² für 0 oder NR¹¹ steht (wobei R¹¹ für Wasserstoff, C₁₋₃-Alkyl oder C₁₋₃-Alkoxy-C₂₋₃-alkyl steht) und X³ für O, NR¹², S, SO oder SO₂ steht (wobei R¹² für Wasserstoff, C₁₋₃-Alkyl oder C₁₋₃-Alkoxy-C₂₋₃-alkyl steht));
3) C₁₋₂-Alkyl-X⁴-C₂₋₃-alkyl-X⁵-C₁₋₃-alkyl (wobei X⁴ und X⁵ unabhängig voneinander für 0, S, SO, SO₂ oder NR¹³ stehen (wobei R¹³ für Wasserstoff, C₁₋₃-Alkyl oder C₁₋₃-Alkoxy-C₂₋₃-alkyl steht)); und
4) C₁₋₃-Alkyl-X⁶-C₁₋₃-alkyl (wobei X⁶ für O, S, SO, SO₂ oder NR¹⁴ steht (wobei R¹⁴ für Wasserstoff, C₁₋₃-Alkyl oder C₁₋₃-Alkoxy-C₂₋₃-alkyl steht));
R³ für Hydroxy, Halogen, Nitro, Fluormethyl, Difluormethyl, Trifluormethyl, Fluormethoxy, Difluormethoxy, Trifluormethoxy, C₁₋₃-Alkyl, Cyano, Amino, oder R¹⁵X⁷ steht,
wobei X⁷ für eine direkte Bindung, 0, CH₂, S, SO, SO₂, NR¹⁶CO, CONR¹⁷, SO₂NR¹⁸, NR¹⁹SO₂ oder NR²⁰ steht (wobei R¹⁶, R¹⁷, R¹⁸, R¹⁹ und R²⁰ jeweils unabhängig voneinander für Wasserstoff, C₁₋₃-Alkyl oder C₁₋₃-Alkoxy-C₂₋₃-alkyl stehen); und
R¹⁵ aus einer der folgenden Gruppen ausgewählt ist:
1) Wasserstoff oder C₁₋₅-Alkyl, das durch eine oder mehrere Gruppen unabhängig voneinander ausgewählt aus Hydroxy, Fluor und Amino substituiert sein kann;
2) C₁₋₅-Alkyl-X⁸-COR²¹ (wobei X⁸ für 0 oder NR²² steht (wobei R²² für Wasserstoff, C₁₋₃-Alkyl oder C₁₋₃-Alkoxy-C₂₋₃-alkyl steht) und R²¹ für C₁₋₃-Alkyl, NR²³R²⁴ oder OR²⁵ steht (wobei R²³, R²⁴ und R²⁵ unabhängig voneinander für Wasserstoff, C₁₋₃-Alkyl oder C₁₋₃-Alkoxy-C₂₋₃-alkyl stehen));
3) C₁₋₅-Alkyl-X⁹-R²⁶ (wobei X⁹ für O, S, SO, SO₂, OCO, NR²⁷CO, CONR²⁸, SO₂NR²⁹, NR³⁰SO₂ oder NR³¹ steht (wobei R²⁷, R²⁸, R²⁹, R³⁰ und R³¹ jeweils unabhängig voneinander für Wasserstoff, C₁₋₃-Alkyl oder C₁₋₃-Alkoxy-C₂₋₃-alkyl stehen) und R²⁶ für Wasserstoff, C₁₋₃-Alkyl, Cyclopentyl, Cyclohexyl oder eine 5- oder 6-gliedrige gesättigte heterocyclische Gruppe mit einem oder zwei Heteroatomen unabhängig voneinander ausgewählt aus 0, S und N steht, wobei die C₁₋₃-Alkylgruppe durch einen oder zwei Substituenten unabhängig voneinander ausgewählt aus Oxo, Hydroxy, Halogen und C₁₋₄-Alkoxy substituiert sein kann und wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
4) C₁₋₅-Alkyl-X¹⁰-C₁₋₅-alkyl-X¹¹R³² (wobei X¹⁰ und X¹¹ jeweils unabhängig voneinander für 0, S, SO, SO₂, NR³³CO, CONR³⁴, SO₂NR³⁵, NR³⁶SO₂ oder NR³⁷ stehen (wobei R³³, R³⁴, R³⁵, R³⁶ und R³⁷ jeweils unabhängig voneinander für Wasserstoff, C₁₋₃-Alkyl oder C₁₋₃-Alkoxy-C₂₋₃-alkyl stehen) und R³² für Wasserstoff oder C₁₋₃-Alkyl steht);
5) R³⁸ (wobei R³⁸ für eine 5- oder 6-gliedrige gesättigte heterocyclische Gruppe mit einem oder zwei Heteroatomen unabhängig voneinander ausgewählt aus 0, S und N steht, wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
6) C₁₋₅-Alkyl-R³⁸ (wobei R³⁸ wie oben definiert ist);
7) C₂₋₅-Alkenyl-R³⁸ (wobei R³⁸ wie oben definiert ist);
8) C₂₋₅-Alkinyl-R³⁸ (wobei R³⁸ wie oben definiert ist);
9) R³⁹ (wobei R³⁹ für eine Pyridongruppe, eine Phenylgruppe oder eine 5- oder 6-gliedrige aromatische heterocyclische Gruppe mit 1 bis 3 Heteroatomen unabhängig voneinander ausgewählt aus O, N und S steht, wobei die Pyridongruppe, die Phenylgruppe bzw. die heterocyclische Gruppe bis zu 5 Substituenten unabhängig voneinander ausgewählt aus Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxyl, Trifluormethyl, Cyano, CONR⁴⁰R⁴¹ und NR⁴²COR⁴³ tragen kann (wobei R⁴⁰, R⁴¹, R⁴² und R⁴³ jeweils unabhängig voneinander für Wasserstoff, C₁₋₄-Alkyl oder C₁₋₃-Alkoxy-C₂₋₃alkyl stehen));
10) C₁₋₅-Alkyl-R³⁹ (wobei R³⁹ wie oben definiert ist);
11) C₂₋₅-Alkenyl-R³⁹ (wobei R³⁹ wie oben definiert ist);
12) C₂₋₅-Alkinyl-R³⁹ (wobei R³⁹ wie oben definiert ist);
13) C₁₋₅-Alkyl-X¹²R³⁹ (wobei X¹² für 0, S, SO, S0₂, NR⁴⁴CO, CONR⁴⁵, SO₂NR⁴⁶, NR⁴⁷SO₂ oder NR⁴⁸ steht (wobei R⁴⁴, R⁴⁵, R⁴⁶, R⁴⁷ und R⁴⁸ jeweils unabhängig voneinander für Wasserstoff, C₁₋₃-Alkyl oder C₁₋₃-Alkoxy-C₂₋₃-alkyl stehen) und R³⁹ wie oben definiert ist);
14) C₂₋₅-Alkenyl-X¹³R³⁹ (wobei X¹³ für O, S, SO, SO₂, NR⁴⁹CO, CONR⁵⁰, SO₂NR⁵¹, NR⁵²SO₂ oder NR⁵³ steht (wobei R⁴⁹, R⁵⁰, R⁵¹, R⁵² und R⁵³ jeweils unabhängig voneinander für Wasserstoff, C₁₋₃-Alkyl oder C₁₋₃-Alkoxy-C₂₋₃-alkyl stehen) und R³⁹ wie oben definiert ist);
15) C₂₋₅-Alkinyl-X¹⁴R³⁹ (wobei X¹⁴ für 0, S, SO, SO₂, NR⁵⁴CO, CONR⁵⁵, SO₂NR⁵⁶, NR⁵⁷SO₂ oder NR⁵⁸ steht (wobei R⁵⁴, R⁵⁵, R⁵⁶, R⁵⁷ und R⁵⁸ jeweils unabhängig voneinander für Wasserstoff, C₁₋₃-Alkyl oder C₁₋₃-Alkoxy-C₂₋₃-alkyl stehen) und R³⁹ für wie oben definiert ist);
16) C₁₋₃-Alkyl-X¹⁵-C₁₋₃-alkyl-R³⁹ (wobei X¹⁵ für O, S, SO, SO₂, NR⁵⁹CO, CONR⁶⁰, SO₂NR⁶¹, NR⁶²SO₂ oder NR⁶³ steht (wobei R⁵⁹, R⁶⁰, R⁶¹, R⁶² und R⁶³ jeweils unabhängig für Wasserstoff, C₁₋₃-Alkyl oder C₁₋₃-Alkoxy-C₂₋₃-alkyl stehen) und R³⁹ wie oben definiert ist); und
17) C₁₋₃-Alkyl-X¹⁵-C₁₋₃-alkyl-R³⁸ (wobei X¹⁵ und R³⁸ wie oben definiert sind);
n für 0, 1, 2 oder 3 steht, wobei Z für einen 6-gliedrigen heterocyclischen Ring steht und n für 0, 1 oder 2 steht, wobei Z für einen 5-gliedrigen heterocyclischen Ring steht;
m für 0, 1, 2, 3 oder 4 steht;
als eine freie Base oder pharmazeutisch annehmbare Salze davon zur Herstellung eines Medikaments zur Behandlung und/oder Prävention von Alzheimer-Krankheit, frontotemporaler Demenz vom Parkinson-Typ, Guam-Parkinsondemenz-Komplex, HIV-Demenz, Prädemenzzuständen, vaskulärer Demenz, Demenz mit Lewy-Körperchen, frontotemporaler Demenz und Dementia pugilistica.

2. Verwendung einer Verbindung der Formel I 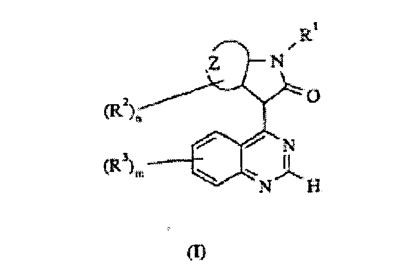 wobei
Ring Z für einen 5- oder 6-gliedrigen heterocyclischen Ring mit 1 bis 3 Heteroatomen unabhängig voneinander ausgewählt aus 0, N und S, jedoch nicht mit mehr als 2 Stickstoffatomen, steht;
R¹ für Wasserstoff oder C₁₋₃-Alkyl steht;
R² für Hydroxy, Halogen, Fluormethyl, Difluormethyl, Trifluormethyl, Fluormethoxy, Difluormethoxy, Trifluormethoxy, 2,2,2-Trifluorethyl, Cyano, Amino, Nitro, C₁₋₃-Alkyl, C₁₋₃-Alkoxy, C₁₋₃-Alkanoyloxy, C₂₋₄-Alkanoyl, C₁₋₄-Alkanoylamino, C₁₋₄-Alkoxycarbonyl, C₁₋₄-Alkylthio, C₁₋₄-Alkylsulfinyl, C₁₋₄-Alkylsulfonyl, Carbamoyl, *N*-C₁₋₄-Alkylcarbamoyl, *N,N*-Di(C₁₋₄-alkyl)carbamoyl, Aminosulfonyl, *N*-C₁₋₄-Alkylaminosulfonyl, *N,N-*Di (C₁₋₄-alkyl)aminosulfonyl oder C₁₋₄-Alkylsulfonylamino steht, oder
R² ausgewählt ist aus einer der folgenden Gruppen:
1) R⁴X¹, wobei X¹ für eine direkte Bindung, O, NR⁵, C₁₋₃-Alkyl, C₂₋₄-Alkanoyl, CONR⁶R⁷, SO₂NR⁸R⁹ oder SO₂R¹⁰ steht (wobei R⁵, R⁶ und R⁸ jeweils unabhängig voneinander für Wasserstoff oder C₁₋₂-Alkyl stehen und R⁷, R⁹ und R¹⁰ jeweils unabhängig voneinander für C₁₋₄-Alkyl stehen und wobei R⁴ an R⁷, R⁹ oder R¹⁰ gebunden ist); und
R⁴ für Phenyl oder eine 5- oder 6-gliedrige heterocyclische Gruppe mit einem oder zwei Heteroatomen unabhängig voneinander ausgewählt aus O, S und N steht, wobei die heterocyclische Gruppe gesättigt oder ungesättigt sein kann und wobei die Phenylgruppe bzw. die heterocyclische Gruppe durch einen oder zwei Substituenten unabhängig voneinander ausgewählt aus Oxo, Hydroxy, Halogen, C₁₋₃-Alkyl, C₁₋₃-Alkoxy, C₁₋₃-Alkanoyloxy, Trifluormethyl, Cyano, Amino, Nitro und C₁₋₄-Alkoxycarbonyl substituiert sein kann;
2) X²-C₂₋₄-Alkyl-X³-C₁₋₃-alkyl (wobei X² für O oder NR¹¹ steht (wobei R¹¹ für Wasserstoff, C₁₋₃-Alkyl oder C₁₋₃-Alkoxy-C₂₋₃-alkyl steht) und X³ für 0, NR¹², S, SO oder SO₂ steht (wobei R¹² für Wasserstoff, C₁₋₃-Alkyl oder C₁₋₃-Alkoxy-C₂₋₃-alkyl steht));
3) C₁₋₂-Alkyl-X⁴-C₂₋₃-alkyl-X⁵-C₁₋₃-alkyl (wobei X⁴ und X⁵ unabhängig voneinander für O, S, SO, SO₂ oder NR¹³ stehen (wobei _{R}¹³ für Wasserstoff, C₁₋₃-Alkyl oder C₁₋₃-Alkoxy-C₂₋₃-alkyl steht)); und
4) C₁₋₃-Alkyl-X⁶-C₁₋₃-alkyl (wobei X⁶ für O, S, SO, SO₂ oder NR¹⁴ steht (wobei R¹⁴ für Wasserstoff, C₁₋₃-Alkyl oder C₁₋₃-Alkoxy-C₂₋₃-alkyl steht));
R³ für Hydroxy, Halogen, Nitro, Fluormethyl, Difluormethyl, Trifluormethyl, Fluormethoxy, Difluormethoxy, Trifluormethoxy, C₁₋₃-Alkyl, Cyano, Amino, oder R¹⁵X⁷ steht,
wobei X⁷ für eine direkte Bindung, O, CH₂, S, SO, SO₂, NR¹⁶CO, CONR¹⁷, SO₂NR¹⁸, NR¹⁹SO₂ oder NR²⁰ steht (wobei R¹⁶, R¹⁷, R¹⁸, R¹⁹ und R²⁰ jeweils unabhängig voneinander für Wasserstoff, C₁₋₃-Alkyl oder C₁₋₃-Alkoxy-C₂₋₃-alkyl stehen); und
R¹⁵ aus einer der folgenden Gruppen ausgewählt ist:
1) Wasserstoff oder C₁₋₅-Alkyl, das durch eine oder mehrere Gruppen unabhängig voneinander ausgewählt aus Hydroxy, Fluor und Amino substituiert sein kann;
2) C₁₋₅-Alkyl-X⁸-COR²¹ (wobei X⁸ für O oder NR²² steht (wobei R²² für Wasserstoff, C₁₋₃-Alkyl oder C₁₋₃-Alkoxy-C₂₋₃-alkyl steht) und R²¹ für C₁₋₃-Alkyl, NR²³R²⁴ oder OR²⁵ steht (wobei R²³, R²⁴ und R²⁵ unabhängig voneinander für Wasserstoff, C₁₋₃-Alkyl oder C₁₋₃-Alkoxy-C₂₋₃-alkyl stehen));
3) C₁₋₅-Alkyl-X⁹-R²⁶ (wobei X⁹ für O, S, SO, SO₂, OCO, NR²⁷CO, CONR²⁸, SO₂NR²⁹, NR³⁰SO₂ oder NR³¹ steht (wobei R²⁷, R²⁸, R²⁹, R³⁰ und R³¹ jeweils unabhängig voneinander für Wasserstoff, C₁₋₃-Alkyl oder C₁₋₃-Alkoxy-C₂₋₃-alkyl stehen) und R²⁶ für Wasserstoff, C₁₋₃-Alkyl, Cyclopentyl, Cyclohexyl oder eine 5- oder 6-gliedrige gesättigte heterocyclische Gruppe mit einem oder zwei Heteroatomen unabhängig voneinander ausgewählt aus O, S und N steht, wobei die C₁₋₃-Alkylgruppe durch einen oder zwei Substituenten unabhängig voneinander ausgewählt aus Oxo, Hydroxy, Halogen und C₁₋₄-Alkoxy substituiert sein kann und wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
4) C₁₋₅-Alkyl-X¹⁰-C₁₋₅-alkyl-X¹¹R³² (wobei X¹⁰ und X¹¹ jeweils unabhängig voneinander für O, S, SO, SO₂, NR³³CO, CONR³⁴, SO₂NR³⁵, NR³⁶SO₂ oder NR³⁷ stehen (wobei R³³, R³⁴, R³⁵, R³⁶ und R³⁷ jeweils unabhängig voneinander für Wasserstoff, C₁₋₃-Alkyl oder C₁₋₃-Alkoxy-C₂₋₃-alkyl stehen) und R³² für Wasserstoff oder C₁₋₃-Alkyl steht);
5) R³⁸ (wobei R³⁸ für eine 5- oder 6-gliedrige gesättigte heterocyclische Gruppe mit einem oder zwei Heteroatomen unabhängig voneinander ausgewählt aus 0, S und N steht, wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
6) C₁₋₅-Alkyl-R³⁸ (wobei R³⁸ wie oben definiert ist);
7) C₂₋₅₋Alkenyl-R³⁸ (wobei R³⁸ wie oben definiert ist);
8) C₂₋₅-Alkinyl-R³⁸ (wobei R³⁸ wie oben definiert ist);
9) R³⁹ (wobei R³⁹ für eine Pyridongruppe, eine Phenylgruppe oder eine 5- oder 6-gliedrige aromatische heterocyclische Gruppe mit 1 bis 3 Heteroatomen unabhängig voneinander ausgewählt aus O, N und S steht, wobei die Pyridongruppe, die Phenylgruppe bzw. die heterocyclische Gruppe bis zu 5 Substituenten unabhängig voneinander ausgewählt aus Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxyl, Trifluormethyl, Cyano, CONR⁴⁰R⁴¹ und NR⁴²COR⁴³ tragen kann (wobei R⁴⁰, R⁴¹, R⁴² und R⁴³ jeweils unabhängig voneinander für Wasserstoff, C₁₋₄-Alkyl oder C₁₋₃-Alkoxy-C₂₋₃-alkyl stehen));
10) C₁₋₅-Alkyl-R³⁹ (wobei R³⁹ wie oben definiert ist);
11) C₂₋₅-Alkenyl-R³⁹ (wobei R³⁹ wie oben definiert ist);
12) C₂₋₅-Alkinyl-R³⁹ (wobei R³⁹ wie oben definiert ist);
13) C₁₋₅-Alkyl-X¹²R³⁹ (wobei X¹² für O, S, SO, SO₂, NR⁴⁴CO, CONR⁴⁵, SO₂NR⁴⁶, NR⁴⁷SO₂ oder NR⁴⁸ steht (wobei R⁴⁴, R⁴⁵, R⁴⁶, R⁴⁷ und R⁴⁸ jeweils unabhängig voneinander für Wasserstoff, C₁₋₃-Alkyl oder C₁₋₃-Alkoxy-C₂₋₃-alkyl stehen) und R³⁹ wie oben definiert ist);
14) C₂₋₅-Alkenyl-X¹³R³⁹ (wobei X¹³ für O, S, SO, SO₂, NR⁹⁹CO, CONR⁵⁰, SO₂NR⁵¹, NR⁵²SO₂ oder NR⁵³ steht (wobei R⁴⁹, R⁵⁰, R⁵¹, R⁵² und R⁵³ jeweils unabhängig voneinander für Wasserstoff, C₁₋₃-Alkyl oder C₁₋₃-Alkoxy-C₂₋₃-alkyl stehen) und R³⁹ für wie oben definiert ist);
15) C₂₋₅-Alkinyl-X¹⁴R³⁹ (wobei X¹⁴ für O, S, SO, SO₂, NR⁵⁴CO, CONR⁵⁵, SO₂NR⁵⁶, NR⁵⁷SO₂ oder NR⁵⁸ steht (wobei R⁵⁴, R⁵⁵, R⁵⁶, R⁵⁷ und R⁵⁸ jeweils unabhängig voneinander für Wasserstoff, C₁₋₃-Alkyl oder C₁₋₃-Alkoxy-C₂₋₃-alkyl stehen) und R³⁹ wie oben definiert ist);
16) C₁₋₃Alkyl-X¹⁵-C₁₋₃alkyl-R³⁹ (wobei X¹⁵ für O, S, SO, SO₂, NR⁵⁹CO, CONR⁶⁰, SO₂NR⁶¹, NR⁶²SO₂ oder NR⁶³ steht (wobei R⁵⁹, R⁶⁰, R⁶¹, R⁶² und R⁶³ jeweils unabhängig für Wasserstoff, C₁₋₃-Alkyl oder C₁₋₃-Alkoxy-C₂₋₃-alkyl stehen) und R³⁹ wie oben definiert ist); und
17) C₁₋₃-Alkyl-X¹⁵-C₁₋₃-alkyl-R³⁸ (wobei X¹⁵ und R³⁸ wie oben definiert sind);
n für 0, 1, 2 oder 3 steht, wobei Z für einen 6-gliedrigen heterocyclischen Ring steht und n für 0, 1 oder 2 steht, wobei Z für einen 5-gliedrigen heterocyclischen Ring steht;
m für 0, 1, 2, 3 oder 4 steht;
als eine freie Base oder pharmazeutisch annehmbare Salze davon zur Herstellung eines Medikaments zur Behandlung und/oder Prävention von amyotropher Lateralsklerose, kortikobasaler Degeneration, Down-Syndrom, Chorea Huntington, Parkinson-Krankheit, postencephalischem Parkinsonismus, progressiver supranukleärer Lähmung, Pick-Krankheit, Niemann-Pick-Krankheit, Schlaganfall, Kopftrauma und anderen chronischen neurodegenerativen Erkrankungen, manischdepressiver Psychose, affektiven Störungen, Depression, Schizophrenie und kognitiven Störungen.

3. Verwendung einer Verbindung der Formel I 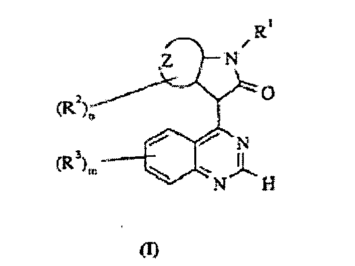 wobei:
Ring Z für einen 5- oder 6-gliedrigen heterocyclischen Ring mit 1 bis 3 Heteroatomen unabhängig voneinander ausgewählt aus O, N und S, jedoch nicht mit mehr als 2 Stickstoffatomen, steht;
R¹ für Wasserstoff oder C₁₋₃-Alkyl steht;
R² für Hydroxy, Halogen, Fluormethyl, Difluormethyl, Trifluormethyl, Fluormethoxy, Difluormethoxy, Trifluormethoxy, 2,2,2-Trifluorethyl, Cyano, Amino, Nitro, C₁₋₃-Alkyl, C₁₋₃-Alkoxy, C₁₋₃-Alkanoyloxy, C₂₋₄-Alkanoyl, C₁₋₄-Alkanoylamino, C₁₋₄-Alkoxycarbonyl, C₁₋₄-Alkylthio, C₁₋₉-Alkylsulfinyl, C₁₋₄-Alkylsulfonyl, Carbamoyl, *N*-C₁₋₄-Alkylcarbamoyl, *N*,*N*-Di(C₁₋₄-alkyl)carbamoyl, Aminosulfonyl, *N*-C₁₋₄-Alkylaminosulfonyl, *N,N-*Di(C₁₋₄-alkyl)aminosulfonyl oder C₁₋₄-Alkylsulfonylamino steht, oder
R² ausgewählt ist aus einer der folgenden Gruppen:
1) R⁴X¹, wobei X¹ für eine direkte Bindung, O, NR⁵, C₁₋₃-Alkyl, C₂₋₄-Alkanoyl, CONR⁶R⁷, SO₂NR⁸R⁹ oder SO₂R¹⁰ steht (wobei R⁵, R⁶ und R⁸ jeweils unabhängig voneinander für Wasserstoff oder C₁₋₂-Alkyl stehen und R⁷, R⁹ und R¹⁰ jeweils unabhängig voneinander für C₁₋₄-Alkyl stehen und wobei R⁴ an R⁷, R⁹ oder R¹⁰ gebunden ist); und
R⁴ für Phenyl oder eine 5- oder 6-gliedrige heterocyclische Gruppe mit einem oder zwei Heteroatomen unabhängig voneinander ausgewählt aus O, S und N steht, wobei die heterocyclische Gruppe gesättigt oder ungesättigt sein kann und wobei die Phenylgruppe bzw. die heterocyclische Gruppe durch einen oder zwei Substituenten unabhängig voneinander ausgewählt aus Oxo, Hydroxy, Halogen, C₁₋₃-Alkyl, C₁₋₃-Alkoxy, C₁₋₃-Alkanoyloxy, Trifluormethyl, Cyano, Amino, Nitro und C₁₋₄-Alkoxycarbonyl substituiert sein kann;
2) X²-C₂₋₄-Alkyl-X³-C₁₋₃-alkyl (wobei X² für O oder NR¹¹ steht (wobei R¹¹ für Wasserstoff, C₁₋₃-Alkyl oder C₁₋₃-Alkoxy-C₂₋₃-alkyl steht) und X³ für 0, NR¹², S, SO oder SO₂ steht (wobei R¹² für Wasserstoff, C₁₋₃-Alkyl oder C₁₋₃-Alkoxy-C₂₋₃-alkyl steht));
3) C₁₋₂-Alkyl-X⁴-C₂₋₃-alkyl-X⁵-C₁₋₃-alkyl (wobei X⁴ und X⁵ unabhängig voneinander für O, S, SO, SO₂ oder NR¹³ stehen (wobei R¹³ für Wasserstoff, C₁₋₃-Alkyl oder C₁₋₃-Alkoxy-C₂₋₃-alkyl steht)); und
4) C₁₋₃-Alkyl-X⁶-C₁₋₃-alkyl (wobei X⁶ für 0, S, SO, SO₂ oder NR¹⁴ steht (wobei R¹⁴ für Wasserstoff, C₁₋₃-Alkyl oder C₁₋₃-Alkoxy-C₂₋₃-alkyl steht));
R³ für Hydroxy, Halogen, Nitro, Fluormethyl, Difluormethyl, Trifluormethyl, Fluormethoxy, Difluormethoxy, Trifluormethoxy, C₁₋₃-Alkyl, Cyano, Amino, oder R¹⁵X⁷ steht,
wobei X⁷ für eine direkte Bindung, O, CH₂, S, SO, SO₂, NR¹⁶CO, CONR¹⁷, SO₂NR¹⁸, NR¹⁹SO₂ oder NR²⁰ steht (wobei R¹⁶, R¹⁷, R¹⁸, R¹⁹ und R²⁰ jeweils unabhängig voneinander für Wasserstoff, C₁₋₃-Alkyl oder C₁₋₃-Alkoxy-C₂₋₃-alkyl stehen); und
R¹⁵ aus einer der folgenden Gruppen ausgewählt ist:
1) Wasserstoff oder C₁₋₅-Alkyl, das durch eine oder mehrere Gruppen unabhängig voneinander ausgewählt aus Hydroxy, Fluor und Amino substituiert sein kann;
2) C₁₋₅-Alkyl-X⁸-COR²¹ (wobei X⁸ für O oder NR²² steht (wobei R²² für Wasserstoff, C₁₋₃-Alkyl oder C₁₋₃-Alkoxy-C₂₋₃-alkyl steht) und R²¹ für C₁₋₃-Alkyl, NR²³R²⁴ oder OR²⁵ steht (wobei R²³, R²⁴ und R²⁵ unabhängig voneinander für Wasserstoff, C₁₋₃-Alkyl oder C₁₋₃-Alkoxy-C₂₋₃-alkyl stehen));
3) C₁₋₅-Alkyl-X⁹-R²⁶ (wobei X⁹ für O, S, SO, SO₂, OCO, NR²⁷CO, CONR²⁸, SO₂NR²⁹, NR³⁰SO₂ oder NR³¹ steht (wobei R²⁷, R²⁸, R²⁹, R³⁰ und R³¹ jeweils unabhängig voneinander für Wasserstoff, C₁₋₃-Alkyl oder C₁₋₃-Alkoxy-C₂₋₃-alkyl stehen) und R²⁶ für Wasserstoff, C₁₋₃-Alkyl, Cyclopentyl, Cyclohexyl oder eine 5- oder 6-gliedrige gesättigte heterocyclische Gruppe mit einem oder zwei Heteroatomen unabhängig voneinander ausgewählt aus 0, S und N steht, wobei die C₁₋₃-Alkylgruppe durch einen oder zwei Substituenten unabhängig voneinander ausgewählt aus Oxo, Hydroxy, Halogen und C₁₋₄-Alkoxy substituiert sein kann und wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
4) C₁₋₅-Alkyl-X¹⁰-C₁₋₅-alkyl-X¹¹R³² (wobei X¹⁰ und X¹¹ jeweils unabhängig voneinander für O, S, SO, SO₂, NR³³CO, CONR³⁴, SO₂NR³⁵, NR³⁶SO₂ oder NR³⁷ stehen (wobei R³³, R³⁴, R³⁵ R³⁶ und R³⁷ jeweils unabhängig voneinander für Wasserstoff, C₁₋₃-Alkyl oder C₁₋₃-Alkoxy-C₂₋₃-alkyl stehen) und R³² für Wasserstoff oder C₁₋₃-Alkyl steht);
5) R³⁸ (wobei R³⁸ für eine 5- oder 6-gliedrige gesättigte heterocyclische Gruppe mit einem oder zwei Heteroatomen unabhängig voneinander ausgewählt aus O, S und N steht, wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
6) C₁₋₅-Alkyl-R³⁸ (wobei R³⁸ wie oben definiert ist);
7) C₂₋₅-Alkenyl-R³⁸ (wobei R³⁸ wie oben definiert ist);
8) C₂₋₅-Alkinyl-R³⁸ (wobei R³⁸ wie oben definiert ist);
9) R³⁹ (wobei R³⁹ für eine Pyridongruppe, eine Phenylgruppe oder eine 5- oder 6-gliedrige aromatische heterocyclische Gruppe mit 1 bis 3 Heteroatomen unabhängig voneinander ausgewählt aus 0, N und S steht, wobei die Pyridongruppe, die Phenylgruppe bzw. die heterocyclische Gruppe bis zu 5 Substituenten unabhängig voneinander ausgewählt aus Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxyl, Trifluormethyl, Cyano, CONR⁴⁰R⁴¹ und NR⁴²COR⁴³ tragen kann (wobei R⁴⁰, R⁴¹, R⁴² und R⁴³ jeweils unabhängig voneinander für Wasserstoff, C₁₋₄-Alkyl oder C₁₋₃-Alkoxy-C₂₋₃alkyl stehen));
10) C₁₋₅-Alkyl-R³⁹ (wobei R³⁹ wie oben definiert ist);
11) C₂₋₅-Alkenyl-R³⁹ (wobei R³⁹ wie oben definiert ist);
12) C₂₋₅-Alkinyl-R³⁹ (wobei R³⁹ wie oben definiert ist);
13) C₁₋₅-Alkyl-X¹²R³⁹ (wobei X¹² für 0, S, SO, SO₂, NR⁴⁴CO, CONR⁴⁵, SO₂NR⁴⁶, NR⁴⁷SO₂ oder NR⁴⁸ steht (wobei R⁴⁴, R⁴⁵, R⁴⁶, R⁴⁷ und R⁴⁸ jeweils unabhängig voneinander für Wasserstoff, C₁₋₃-Alkyl oder C₁₋₃-Alkoxy-C₂₋₃-alkyl stehen) und R³⁹ wie oben definiert ist);
14) C₂₋₅-Alkenyl-X¹³R³¹ (wobei X¹³ für O, S, SO, SO₂, NR⁴⁹CO, CONR⁵⁰, SO₂NR⁵¹, NR⁵²SO₂ oder NR⁵³ steht (wobei R⁴⁹, R⁵⁰, R⁵¹, R⁵² und R⁵³ jeweils unabhängig voneinander für Wasserstoff, C₁₋₃-Alkyl oder C₁₋₃-Alkoxy-C₂₋₃-alkyl stehen) und R³⁹ wie oben definiert ist);
15) C₂₋₅-Alkinyl-X¹⁴R³⁹ (wobei X¹⁴ für 0, S, SO, SO₂, NR⁵⁹CO, CONR⁵⁵, SO₂NR⁵⁶, NR⁵⁷SO₂ oder NR⁵⁸ steht (wobei R⁵⁴, R⁵⁵, R⁵⁶, R⁵⁷ und R⁵⁸ jeweils unabhängig voneinander für Wasserstoff, C₁₋₃-Alkyl oder C₁₋₃-Alkoxy-C₂₋₃-alkyl stehen) und R³⁹ wie oben definiert ist);
16) C₁₋₃-Alkyl-X¹⁵-C₁₋₃-alkyl-R³⁹ (wobei X¹⁵ für O, S, SO, SO₂, NR⁵⁹CO, CONR⁶⁰, SO₂NR⁶¹, NR⁶²SO₂ oder NR⁶³ steht (wobei R⁵⁹, R⁶⁰, R⁶¹, R⁶² und R⁶³ jeweils unabhängig für Wasserstoff, C₁₋₃-Alkyl oder C₁₋₃-Alkoxy-C₂₋₃-alkyl stehen) und R³⁹ wie oben definiert ist); und
17) C₁₋₃-Alkyl-X¹⁵-C₁₋₃-alkyl-R³⁸ (wobei X¹⁵ und R³⁸ wie oben definiert sind);
n für 0, 1, 2 oder 3 steht, wobei Z für einen 6-gliedrigen heterocyclischen Ring steht und n für 0, 1 oder 2 steht, wobei Z für einen 5-gliedrigen heterocyclischen Ring steht;
m für 0, 1, 2, 3 oder 4 steht;
als eine freie Base oder pharmazeutisch annehmbare Salze davon zur Herstellung eines Medikaments zur Behandlung und/oder Prävention von schwacher kognitiver Störung, altersbedingter Gedächtnisstörung, altersbedingter kognitiver Abnahme, kognitiver Störung ohne Demenz, schwacher kognitiver Abnahme, schwacher neurokognitiver Abnahme, Vergeßlichkeit im hohen Alter, Gedächtnisstörung und kognitiver Störung.

4. Verwendung nach einem der Ansprüche 1-3, wobei Z für einen 6-gliedrigen heterocyclischen Ring mit 1 oder 2 Stickstoffatomen steht und R¹ für Wasserstoff steht.

5. Verwendung nach einem der Ansprüche 1 bis 4, wobei
R² für Halogen, C₁₋₃-Alkyl, Trifluormethyl, Cyano, Carbamoyl, *N*-C₁₋₄-Alkylcarbamoyl, Aminosulfonyl oder eine Gruppe R⁴X¹ steht,
wobei X¹ für CONR⁶R⁷ steht (wobei R⁶ für Wasserstoff oder C₁₋₂-Alkyl steht und R⁷ für C₁₋₄-Alkyl steht und
wobei R⁴ an R⁷ gebunden ist); und
n für 0 oder 1 steht.

6. Verwendung nach einem der Ansprüche 1-5,
wobei R³ für R¹⁵X⁷ steht,
wobei X⁷ für O steht; und
R¹⁵ ausgewählt ist aus einer der folgenden Gruppen:
1) Wasserstoff oder C₁₋₅-Alkyl;
3) C₁₋₅-Alkyl-X⁹R²⁶ (wobei X⁹ für O steht (wobei R²⁶ für Wasserstoff oder C₁₋₃-Alkyl steht));
4) C₁₋₅-Alkyl-X¹⁰-C₁₋₅-alkyl-X¹¹R³² (wobei X¹⁰ und X¹¹ für O stehen und R³² für Wasserstoff oder C₁₋₃-Alkyl steht);
6) C₁₋₅-Alkyl-R³⁸ (wobei R³⁸ für eine 6-gliedrige gesättigte heterocyclische Gruppe mit einem oder zwei Heteroatomen unabhängig voneinander ausgewählt aus O, S und N steht, wobei die heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
7) C₂₋₅-Alkenyl-R³⁸ (wobei R³⁸ wie oben definiert ist);
10) C₁₋₅-Alkyl-R³⁹ (wobei R³⁹ für eine 5- oder 6-gliedrige aromatische heterocyclische Gruppe mit 1 oder 3 Heteroatomen unabhängig voneinander ausgewählt aus O, N und S steht, wobei die heterocyclische Gruppe bis zu 4 Substituenten unabhängig voneinander ausgewählt aus Hydroxy, Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxy, Trifluormethyl, Cyano, CONR⁴⁰R⁴¹ und NR⁴²COR⁴³ tragen kann (wobei R⁴⁰, R⁴¹, R⁴² und R⁴³ jeweils für Wasserstoff, C₁₋₄-Alkyl oder C₁₋₃-Alkoxy-C₂₋₃-alkyl stehen));
13) C₁₋₅-Alkyl-X¹²R³⁹ (wobei X¹² für O steht und R³⁹ wie oben definiert ist);
m für 0, 1 oder 2 steht.

7. Verwendung nach einem der Ansprüche 1-6, wobei die Verbindungen ausgewählt sind aus
4-(7-Azaoxindol-3-yl)-6-methoxy-7-(2-methoxyethoxy)chinazolin,
4-(7-Azaoxindol-3-yl)-6-methoxy-7-(3-morpholinopropoxy)chinazolin,
4-(7-Azaoxindol-3-yl)-7-(2-imidazol-1-yl)ethoxy)-6-methoxychinazolin,
4-(5,7-Diaza-6-methyloxindol-3-yl)-7-(3-morpholinopropoxy)chinazolin,
4-(7-Aza-6-chloroxindol-3-yl)-7-(3-morpholinopropoxy)chinazolin,
4-(5,7-Diaza-6-methyloxindol-3-yl)-6-methoxy-7-(2-(1,2,3-triazol-1-yl)ethoxy)chinazolin,
4-(5,7-Diaza-6-methyloxind-ol-3-yl)-7-(2-(2-methoxyethoxy)ethoxy)chinazolin,
4-(7-Azaoxindol-3-yl)-7-(3-morpholinopropoxy)chinazolin,
4-(7-Azaoxindol-3-yl)-7-(2-(2-methoxyethoxy)ethoxy)chinazolin,
4-(7-Azaoxindol-3-yl)-7-(4-morpholinobut-2-en-1-yloxy)chinazolin,
4-(5,7-Diaza-6-methyloxindol-3-yl)-6-methoxy-7-(2-(4-pyridyloxy)ethoxy)chinazolin,
4-(7-Aza-6-chloroxindol-3-yl)-7-(2-(2-methoxyethoxy)ethoxy)chinazolin und
4-(5,7-Diaza-6-methyloxindol-3-yl)-7-(3-(1,1-dioxothiomorpholino)propoxy)chinazolin;
als eine freie Base oder pharmazeutisch annehmbare Salze davon.

## Revendications

1. Utilisation d'un composé de formule I 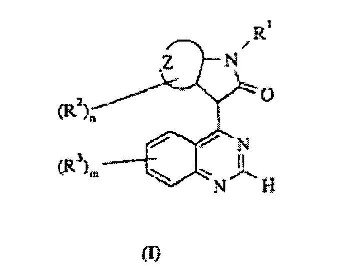 dans laquelle :
le cycle Z est un cycle hétérocyclique à 5 ou 6 chaînons contenant de 1 à 3 hétéroatomes choisis indépendamment parmi O, N et S mais pas plus de 2 atomes d'azote ;
R¹ est hydrogène ou C₁₋₃alkyle ;
R² est hydroxy, halogéno, fluorométhyle, difluorométhyle, trifluorométhyle, fluorométhoxy, difluorométhoxy, trifluorométhoxy, 2,2,2-trifluoroéthyle, cyano, amino, nitro, C₁₋₃alkyle, C₁₋₃alcoxy, C₁₋₃alcanoyloxy, C₂₋₄alcanoyle, C₁₋₄alcanoylamino, C₁₋₄alcoxycarbonyle, C₁₋₄alkylthio, C₁₋₄alkylsulfinyle, C₁₋₄alkylsulfonyle, carbamoyle, *N*-C₁₋₄alkylcarbamoyle, *N,N-*di(C₁₋₄alkyl) carbamoyle, aminosulfonyle, *N*-C₁₋₄alkylaminosulfonyle, *N,N-*di(C₁₋₄alkyl)aminosulfonyle ou C₁₋₄alkylsulfonylamino, ou R² est choisi dans l'un des groupes suivants :
1) R⁴X¹, où X¹ est une liaison directe, O, NR⁵, C₁₋₃alkyle, C₂₋₄alcanoyle, CONR⁶R⁷, SO₂NR⁸R⁹ ou SO₂R¹⁰ (où R⁵, R⁶ et R⁸ représentent chacun indépendamment hydrogène ou C₁₋₂alkyle et R⁷, R⁹ et R¹⁰ représentent chacun indépendamment C₁₋₄alkyle et où R⁴ est lié à R⁷, R⁹ ou R¹⁰) ; et
R⁹ est phényle ou un groupement hétérocyclique à 5 ou 6 chaînons avec un ou deux hétéroatomes choisis indépendamment parmi O, S et N, lequel groupement hétérocyclique peut être saturé ou insaturé et lequel groupement phényle ou hétérocyclique peut être substitué par un ou deux substituants choisis indépendamment parmi oxo, hydroxy, halogéno, C₁₋₃alkyle, C₁₋₃alcoxy, C₁₋₃alcanoyloxy, trifluorométhyle, cyano, amino, nitro et C₁₋₄alcoxycarbonyle ;
2) X²C₂₋₄alkylX³C₁₋₃alkyle (où X² est 0 ou NR¹¹ (où R¹¹ est hydrogène, C₁₋₃alkyle ou C₁₋₃alcoxyC₂₋₃alkyle) et X³ est O, NR¹², S, SO ou SO₂ (où R¹² est hydrogène, C₁₋₃alkyle ou C₁₋₃alcoxyC₂₋₃alkyle)) ;
3) C₁₋₂alkylX⁴C₂₋₃alkylX⁵C₁₋₃alkyle (où X⁴ et X⁵ représentent chacun indépendamment O, S, SO, SO₂ ou NR¹³ (où R¹³ est hydrogène, C₁₋₃alkyle ou C₁₋₃alcoxyC₂₋₃alkyle)); et
4) C₁₋₃alkylX⁶C₁₋₃alkyle (où X⁶ est O, S, SO, SO₂ ou NR¹⁴ (où R¹⁴ est hydrogène, C₁₋₃alkyle ou C₁₋₃alcoxyC₂₋₃alkyle));
R³ est hydroxy, halogéno, nitro, fluorométhyle, difluorométhyle, trifluorométhyle, fluorométhoxy, difluorométhoxy, trifluorométhoxy, C₁₋₃alkyle, cyano, amino, ou R¹⁵X⁷,
où X⁷ est une liaison directe, O, CH₂, S, SO, SO₂, NR¹⁶CO, CONR¹⁷, SO₂NR¹⁸, NR¹⁹SO₂ ou NR²⁰ (où R¹⁶, R¹⁷, R¹⁸, R¹⁹ et R²⁰ représentent chacun indépendamment hydrogène, C₁₋₃alkyle ou C₁₋₃alcoxyC₂₋₃alkyle); et
R¹⁵ est choisi dans l'un des groupes suivants :
1) hydrogène ou C₁₋₅alkyle, qui peut être substitué par un ou plusieurs groupements choisis indépendamment parmi hydroxy, fluoro et amino ;
2) C₁₋₅alkylX⁸COR²¹ (où X⁸ est 0 ou NR²² (où R²² est hydrogène, C₁₋₃alkyle ou C₁₋₃alcoxyC₂₋₃alkyle) et R²¹ est C₁₋₃alkyle, NR²³R²⁴ ou OR²⁵ (où R²³, R²⁴ et R²⁵ représentent chacun indépendamment hydrogène, C₁₋₃alkyle ou C₁₋₃alcoxyC₂₋₃alkyle)) ;
3) C₁₋₅alkylX⁹R²⁶ (où X⁹ est O, S, SO, SO₂, OCO, NR²⁷CO, CONR²⁸, SO₂NR²⁹, NR³⁰SO₂ ou NR³¹ (où R²⁷, R²⁸, R²⁹, R³⁰ et R³¹ représentent chacun indépendamment hydrogène, C₁₋₃alkyle ou C₁₋₃alcoxyC₂₋₃alkyle) et R²⁶ est hydrogène, C₁₋₃alkyle, cyclopentyle, cyclohexyle ou un groupement hétérocyclique saturé à 5 ou 6 chaînons avec un ou deux hétéroatomes choisis indépendamment parmi O, S et N, lequel groupement C₁₋₃alkyle peut être substitué par un ou deux substituants choisis indépendamment parmi oxo, hydroxy, halogéno et C₁₋₄alcoxy et lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
4) C₁₋₅alkylX¹⁰C₁₋₅alkylX¹¹R³² (où X¹⁰ et X¹¹ représentent chacun indépendamment 0, S, SO, S0₂, NR³³CO, CONR³⁴, SO₂NR³⁵, NR³⁶SO₂ ou NR³⁷ (où R³³, R³⁴, R³⁵, R³⁶ et R³⁷ représentent chacun indépendamment hydrogène, C₁₋₃alkyle ou C₁₋₃alcoxyC₂₋₃alkyle) et R³² est hydrogène ou C₁₋₃alkyle) ;
5) R³⁸ (où R³⁸ est un groupement hétérocyclique saturé à 5 ou 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy);
6) C₁₋₅alkylR³⁸ (où R³⁸ est tel que défini précédemment);
7) C₂₋₅alcénylR³⁸ (où R³⁸ est tel que défini précédemment);
8) C₂₋₅alcynylR³⁸ (où R³⁸ est tel que défini précédemment);
9) R³⁹ (où R³⁹ est un groupement pyridone, un groupement phényle ou un groupement hétérocyclique aromatique à 5 ou 6 chaînons avec 1 à 3 hétéroatomes choisis indépendamment parmi 0, N et S, lequel groupement pyridone, phényle ou hétérocyclique peut porter jusqu'à 5 substituants choisis indépendamment parmi hydroxy, halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, trifluorométhyle, cyano, CONR⁴⁰R⁴¹ et NR⁴²COR⁴³ (où R⁴⁰, R⁴¹, R⁴² et R⁴³ représentent chacun indépendamment hydrogène, C₁₋₄alkyle ou C₁₋₃alcoxyC₂₋₃alkyle)) ;
10) C₁₋₅alkylR³⁹ (où R³⁹ est tel que défini précédemment) ;
11) C₂₋₅alcénylR³⁹ (où R³⁹ est tel que défini précédemment) ;
12) C₂₋₅alcynylR³⁹ (où R³⁹ est tel que défini précédemment) ;
13) C₁₋₅alkylX¹²R³⁹ (où X¹² est O, S, SO, SO₂, NR⁴⁴CO, CONR⁴⁵, SO₂NR⁴⁶, NR⁴⁷SO₂ ou NR⁴⁸ (où R⁴⁴, R⁴⁵, R⁴⁶, R⁴⁷ et R⁴⁸ représentent chacun indépendamment hydrogène, C₁₋₃alkyle ou C₁₋₃alcoxyC₂₋₃alkyle) et R³⁹ est tel que défini précédemment) ;
14) C₂₋₅alcénylX¹³R³⁹ (où X¹³ est O, S, SO, SO₂, NR⁴⁹CO, CONR⁵⁰, SO₂NR⁵¹, NR⁵²SO₂ ou NR⁵³ (où R⁴⁹, R⁵⁰, R⁵¹, R⁵² et R⁵³ représentent chacun indépendamment hydrogène, C₁₋₃alkyle ou C₁₋₃alcoxyC₂₋₃alkyle) et R³⁹ est tel que défini précédemment) ;
15) C₂₋₅alcynylX¹⁴R³⁹ (où X¹⁴ est O, S, SO, SO₂, NR⁵⁴CO, CONR⁵⁵, SO₂NR⁵⁶, NR⁵⁷SO₂ ou NR⁵⁸ (où R⁵⁴, R⁵⁵, R⁵⁶, R⁵⁷ et R⁵⁸ représentent chacun indépendamment hydrogène, C₁₋₃alkyle ou C₁₋₃alcoxyC₂₋₃alkyle) et R³⁹ est tel que défini précédemment) ;
16) C₁₋₃alkylX¹⁵C₁₋₃alkylR³⁹ (où X¹⁵ est O, S, SO, SO₂, NR⁵⁹CO, CONR⁶⁰, SO₂NR⁶¹, NR⁶²SO₂ ou NR⁶³ (où R⁵⁹, R⁶⁰, R⁶¹, R⁶² et R⁶³ représentent chacun indépendamment hydrogène, C₁₋₃alkyle ou C₁₋₃alcoxyC₂₋₃alkyle) et R³⁹ est tel que défini précédemment) ; et
17) C₁₋₃alkylX¹⁵C₁₋₃alkylR³⁸ (où X¹⁵ et R³⁸ sont tels que définis précédemment) ;
n est 0, 1, 2 ou 3 lorsque Z est un cycle hétérocyclique à 6 chaînons et n est 0, 1 ou 2 lorsque Z est un cycle hétérocyclique à 5 chaînons;
m est 0, 1, 2, 3 ou 4 ;
sous forme de base libre ou de sels pharmaceutiquement acceptables de celui-ci, dans la fabrication d'un médicament destiné au traitement et/ou à la prévention de la maladie d'Alzheimer, de la démence frontotemporale de type Parkinson, du complexe de la démence de Parkinson de Guam, de la démence liée au VIH, des états de prédémence, de la démence vasculaire, de la démence à corps de Lewy, de la démence frontotemporale et de la démence pugilistique.

2. Utilisation d'un composé de formule I 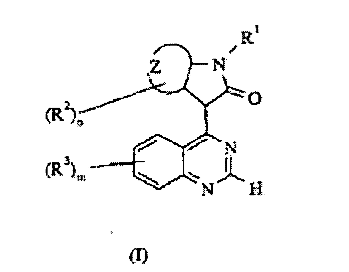 dans laquelle :
le cycle Z est un cycle hétérocyclique à 5 ou 6 chaînons contenant de 1 à 3 hétéroatomes choisis indépendamment parmi 0, N et S mais pas plus de 2 atomes d'azote ;
R¹ est hydrogène ou C₁₋₃alkyle ;
R² est hydroxy, halogéno, fluorométhyle, difluorométhyle, trifluorométhyle, fluorométhoxy, difluorométhoxy, trifluorométhoxy, 2,2,2-trifluoroéthyle, cyano, amino, nitro, C₁₋₃alkyle, C₁₋₃alcoxy, C₁₋₃alcanoyloxy, C₂₋₄alcanoyle, C₁₋₄alcanoylamino, C₁₋₄alcoxycarbonyle, C₁₋₄alkylthio, C₁₋₄alkylsulfinyle, C₁₋₄alkylsulfonyle, carbamoyle, *N*-C₁₋₄alkylcarbamoyle, *N,N*-di(C₁₋₄alkyl)carbamoyle, aminosulfonyle, *N*-C₁₋₄alkylaminosulfonyle, *N,N-*di(C₁₋₄alkyl)aminosulfonyle ou C₁₋₄alkylsulfonylamino, ou
R² est choisi dans l'un des groupes suivants :
1) R⁴X¹, où X¹ est une liaison directe, 0, NR⁵, C₁₋₃alkyle, C₂₋₄alcanoyle, CONR⁶R⁷, SO₂NR⁸R⁹ ou SO₂R¹⁰ (où R⁵, R⁶ et R⁸ représentent chacun indépendamment hydrogène ou C₁₋₂alkyle et R⁷, R⁹ et R¹⁰ représentent chacun indépendamment C₁₋₄alkyle et où R⁴ est lié à R⁷, R⁹ ou R¹⁰) ; et
R⁴ est phényle ou un groupement hétérocyclique à 5 ou 6 chaînons avec un ou deux hétéroatomes choisis indépendamment parmi O, S et N, lequel groupement hétérocyclique peut être saturé ou insaturé et lequel groupement phényle ou hétérocyclique peut être substitué par un ou deux substituants choisis indépendamment parmi oxo, hydroxy, halogéno, C₁₋₃alkyle, C₁₋₃alcoxy, C₁₋₃alcanoyloxy, trifluorométhyle, cyano, amino, nitro et C₁₋₄alcoxycarbonyle ;
2) X²C₂₋₄alkylX³C₁₋₃alkyle (où X² est O ou NR¹¹ (où R¹¹ est hydrogène, C₁₋₃alkyle ou C₁₋₃alcoxyC₂₋₃alkyle) et X³ est O, NR¹², S, SO ou SO₂ (où R¹² est hydrogène, C₁₋₃alkyle ou C₁₋₃alcoxyC₂₋₃alkyle)) ;
3) C₁₋₂alkylX⁴C₂₋₃alkylX⁵C₁₋₃alkyle (où X⁴ et X⁵ représentent chacun indépendamment O, S, SO, SO₂ ou NR¹³ (où R¹³ est hydrogène, C₁₋₃alkyle ou C₁₋₃alcoxyC₂₋₃alkyle)) ; et
4) C₁₋₃alkylX⁶C₁₋₃alkyle (où X⁶ est O, S, SO, SO₂ ou NR¹⁴ (où R¹⁴ est hydrogène, C₁₋₃alkyle ou C₁₋₃alcoxyC₂₋₃alkyle)) ;
R³ est hydroxy, halogéno, nitro, fluorométhyle, difluorométhyle, trifluorométhyle, fluorométhoxy, difluorométhoxy, trifluorométhoxy, C₁₋₃alkyle, cyano, amino, ou R¹⁵X⁷,
où X⁷ est une liaison directe, O, CH₂, S, SO, SO₂, NR¹⁶CO, CONR¹⁷, SO₂NR¹⁸, NR¹⁹SO₂ ou NR²⁰ (où R¹⁶, R¹⁷, R¹⁸, R¹⁹ et R²⁰ représentent chacun indépendamment hydrogène, C₁₋₃alkyle ou C₁₋₃alcoxyC₂₋₃alkyle) ; et
R¹⁵ est choisi dans l'un des groupes suivants :
1) hydrogène ou C₁₋₅alkyle, qui peut être substitué par un ou plusieurs groupements choisis indépendamment parmi hydroxy, fluoro et amino ;
2) C₁₋₅alkylX⁸COR²¹ (où X⁸ est O ou NR²² (où R²² est hydrogène, C₁₋₃alkyle ou C₁₋₃alcoxyC₂₋₃alkyle) et R²¹ est C₁₋₃alkyle, NR²³R²⁴ ou OR²⁵ (où R²³, R²⁴ et R²⁵ représentent chacun indépendamment hydrogène, C₁₋₃alkyle ou C₁₋₃alcoxyC₂₋₃alkyle)) ;
3) C₁₋₅alkylX⁹R²⁶ (où X⁹ est O, S, SO, SO₂, OCO, NR²⁷CO, CONR²⁸, SO₂NR²⁹, NR³⁰SO₂ ou NR³¹ (où R²⁷, R²⁸, R²⁹, R³⁰ et R³¹ représentent chacun indépendamment hydrogène, C₁₋₃alkyle ou C₁₋₃alcoxyC₂₋₃alkyle) et R²⁶ est hydrogène, C₁₋₃alkyle, cyclopentyle, cyclohexyle ou un groupement hétérocyclique saturé à 5 ou 6 chaînons avec un ou deux hétéroatomes choisis indépendamment parmi O, S et N, lequel groupement C₁₋₃alkyle peut être substitué par un ou deux substituants choisis indépendamment parmi oxo, hydroxy, halogéno et C₁₋₄alcoxy et lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy);
4) C₁₋₅alkylX¹⁰C₁₋₅alkylX¹¹R³² (où X¹⁰ et X¹¹ représentent chacun indépendamment O, S, SO, SO₂, NR³³CO, CONR³⁴, SO₂NR³⁵, NR³⁶SO₂ ou NR³⁷ (où R³³, R³⁴, R³⁵, R³⁶ et R³⁷ représentent chacun indépendamment hydrogène, C₁₋₃alkyle ou C₁₋₃alcoxyC₂₋₃alkyle) et R³² est hydrogène ou C₁₋₃alkyle);
5) R³⁸ (où R³⁸ est un groupement hétérocyclique saturé à 5 ou 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy);
6) C₁₋₅alkylR³⁸ (où R³⁸ est tel que défini précédemment);
7) C₂₋₅alcénylR³⁸ (où R³⁸ est tel que défini précédemment) ;
8) C₂₋₅alcynylR³⁸ (où R³⁸ est tel que défini précédemment) ;
9) R³⁹ (où R³⁹ est un groupement pyridone, un groupement phényle ou un groupement hétérocyclique aromatique à 5 ou 6 chaînons avec 1 à 3 hétéroatomes choisis indépendamment parmi 0, N et S, lequel groupement pyridone, phényle ou hétérocyclique peut porter jusqu'à 5 substituants choisis indépendamment parmi hydroxy, halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, trifluorométhyle, cyano, CONR⁴⁰R⁴¹ et NR⁴²COR⁹³ (où R⁴⁰, R⁴¹, R⁴² et R⁴³ représentent chacun indépendamment hydrogène, C₁₋₄alkyle ou C₁₋₃alcoxyC₂₋₃alkyle));
10) C₁₋₅alkylR³⁹ (où R³⁹ est tel que défini précédemment);
11) C₂₋₅alcénylR³⁹ (où R³⁹ est tel que défini précédemment);
12) C₂₋₅alcynylR³⁹ (où R³⁹ est tel que défini précédemment);
13) C₁₋₅alkylX¹²R³⁹ (où X¹² est O, S, SO, SO₂, NR⁴⁴CO, CONR⁴⁵, SO₂NR⁴⁶, NR⁴⁷SO₂ ou NR⁴⁸ (où R⁴⁴, R⁴⁵, R⁴⁶, R⁴⁷ et R⁴⁸ représentent chacun indépendamment hydrogène, C₁₋₃alkyle ou C₁₋₃alcoxyC₂₋₃alkyle) et R³⁹ est tel que défini précédemment) ;
14) C₂₋₅alcénylX¹³R³⁹ (où X¹³ est O, S, SO, SO₂, NR⁴⁹CO, CONR⁵⁰, SO₂NR⁵¹, NR⁵²SO₂ ou NR⁵³ (où R⁴⁹, R⁵⁰, R⁵¹, R⁵² et R⁵³ représentent chacun indépendamment hydrogène, C₁₋₃alkyle ou C₁₋₃alcoxyC₂₋₃alkyle) et R³⁹ est tel que défini précédemment) ;
15) C₂₋₅alcynylX¹⁴R³⁹ (où X¹⁴ est O, S, SO, SO₂, NR⁵⁴CO, CONR⁵⁵, SO₂NR⁵⁶, NR⁵⁷SO₂ ou NR⁵⁸ (où R⁵⁴, R⁵⁵, R⁵⁶, R⁵⁷ et R⁵⁸ représentent chacun indépendamment hydrogène, C₁₋₃alkyle ou C₁₋₃alcoxyC₂₋₃alkyle) et R³⁹ est tel que défini précédemment) ;
16) C₁₋₃alkylX¹⁵C₁₋₃alkylR³⁹ (où X¹⁵ est O, S, SO, SO₂, NR⁵⁹CO, CONR⁶⁰, SO₂NR⁶¹, NR⁶²SO₂ ou NR⁶³ (où R⁵⁹, R⁶⁰, R⁶¹, R⁶² et R⁶³ représentent chacun indépendamment hydrogène, C₁₋₃alkyle ou C₁₋₃alcoxyC₂₋₃alkyle) et R³⁹ est tel que défini précédemment) ; et
17) C₁₋₃alkylX¹⁵C₁₋₃alkylR³⁸ (où X¹⁵ et R³⁸ sont tels que définis précédemment) ;
n est 0, 1, 2 ou 3 lorsque Z est un cycle hétérocyclique à 6 chaînons et n est 0, 1 ou 2 lorsque Z est un cycle hétérocyclique à 5 chaînons ;
m est 0, 1, 2, 3 ou 4 ;
sous forme de base libre ou de sels pharmaceutiquement acceptables de celui-ci, dans la fabrication d'un médicament destiné au traitement et/ou à la prévention de la sclérose latérale amyotrophique, de la dégénérescence corticobasale, du syndrome de Down, de la maladie de Huntington, de la maladie de Parkinson, du syndrome parkinsonien post-encéphalitique, de la paralysie supranucléaire progressive, de la maladie de Pick, de la maladie de Niemann-Pick, de l'accident cérébro-vasculaire, du traumatisme crânien et d'autres maladies neurodégénératives chroniques, d'une maladie bipolaire, de troubles affectifs, de la dépression, de la schizophrénie et de troubles cognitifs.

3. Utilisation d'un composé de formule I 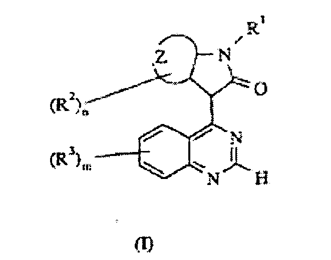 dans laquelle :
le cycle Z est un cycle hétérocyclique à 5 ou 6 chaînons contenant de 1 à 3 hétéroatomes choisis indépendamment parmi O, N et S mais pas plus de 2 atomes d'azote ;
R¹ est hydrogène ou C₁₋₃alkyle ;
R² est hydroxy, halogéno, fluorométhyle, difluorométhyle, trifluorométhyle, fluorométhoxy, difluorométhoxy, trifluorométhoxy, 2,2,2-trifluoroéthyle, cyano, amino, nitro, C₁₋₃alkyle, C₁₋₃alcoxy, C₁₋₃alcanoyloxy, C₂₋₄alcanoyle, C₁₋₄alcanoylamino, C₁₋₄alcoxycarbonyle, C₁₋₄alkylthio, C₁₋₄alkylsulfinyle, C₁₋₄alkylsulfonyle, carbamoyle, *N*-C₁₋₄alkylcarbamoyle, *N,N-*di(C₁₋₄alkyl) carbamoyle, aminosulfonyle, *N*-C₁₋₄alkylaminosulfonyle, *N,N-*di(C₁₋₄alkyl)aminosulfonyle ou C₁₋₄alkylsulfonylamino, ou R² est choisi dans l'un des groupes suivants:
1) R⁴X¹, où X¹ est une liaison directe, O, NR⁵, C₁₋₃alkyle, C₂₋₄alcanoyle, CONR⁶R⁷, SO₂NR⁸R⁹ ou SO₂R¹⁰ (où R⁵, R⁶ et R⁸ représentent chacun indépendamment hydrogène ou C₁₋₂alkyle et R⁷, R⁹ et R¹⁰ représentent chacun indépendamment C₁₋₄alkyle et où R⁴ est lié à R⁷, R⁹ ou R¹⁰) ; et
R⁴ est phényle ou un groupement hétérocyclique à 5 ou 6 chaînons avec un ou deux hétéroatomes choisis indépendamment parmi 0, S et N, lequel groupement hétérocyclique peut être saturé ou insaturé et lequel groupement phényle ou hétérocyclique peut être substitué par un ou deux substituants choisis indépendamment parmi oxo, hydroxy, halogéno, C₁₋₃alkyle, C₁₋₃alcoxy, C₁₋₃alcanoyloxy, trifluorométhyle, cyano, amino, nitro et C₁₋₄alcoxycarbonyle ;
2) X²C₂₋₄alkylX³C₁₋₃alkyle (où X² est O ou NR¹¹ (où R¹¹ est hydrogène, C₁₋₃alkyle ou C₁₋₃alcoxyC₂₋₃alkyle) et X³ est O, NR¹², S, SO ou SO₂ (où R¹² est hydrogène, C₁₋₃alkyle ou C₁₋₃alcoxyC₂₋₃alkyle)) ;
3) C₁₋₂alkylX⁴C₂₋₃alkylX⁵C₁₋₃alkyle (où X⁴ et x⁵ représentent chacun indépendamment O, S, SO, SO₂ ou NR¹³ (où R¹³ est hydrogène, C₁₋₃alkyle ou C₁₋₃alcoxyC₂₋₃alkyle)) ; et
4) C₁₋₃alkylX⁶C₁₋₃alkyle (où X⁶ est O, S, SO, SO₂ ou NR¹⁴ (où R¹⁴ est hydrogène, C₁₋₃alkyle ou C₁₋₃alcoxyC₂₋₃alkyle));
R³ est hydroxy, halogéno, nitro, fluorométhyle, difluorométhyle, trifluorométhyle, fluorométhoxy, difluorométhoxy, trifluorométhoxy, C₁₋₃alkyle, cyano, amino, ou R¹⁵X⁷,
où X⁷ est une liaison directe, 0, CH₂, S, SO, SO₂, NR¹⁶CO, CONR¹⁷, SO₂NR¹⁸, NR¹⁹SO₂ ou NR²⁰ (où R¹⁶, R¹⁷, R¹⁸, R¹⁹ et R²⁰ représentent chacun indépendamment hydrogène, C₁₋₃alkyle ou C₁₋₃alcoxyC₂₋₃alkyle) ; et R¹⁵ est choisi dans l'un des groupes suivants :
1) hydrogène ou C₁₋₅alkyle, qui peut être substitué par un ou plusieurs groupements choisis indépendamment parmi hydroxy, fluoro et amino ;
2) C₁₋₅alkylX⁸COR²¹ (où X⁸ est O ou NR²² (où R²² est hydrogène, C₁₋₃alkyle ou C₁₋₃alcoxyC₂₋₃alkyle) et R²¹ est C₁₋₃alkyle, NR²³R²⁴ ou OR²⁵ (où R²³, R²⁴ et R²⁵ représentent chacun indépendamment hydrogène, C₁₋₃alkyle ou C₁₋₃alcoxyC₂₋₃alkyle)) ;
3) C₁₋₅alkylX⁹R²⁶ (où X⁹ est O, S, SO, SO₂, OCO, NR²⁷CO, CONR²⁸, SO₂NR²⁹, NR³⁰SO₂ ou NR³¹ (où R²⁷, R²⁸, R²⁹, R³⁰ et R³¹ représentent chacun indépendamment hydrogène, C₁₋₃alkyle ou C₁₋₃alcoxyC₂₋₃alkyle) et R²⁶ est hydrogène, C₁₋₃alkyle, cyclopentyle, cyclohexyle ou un groupement hétérocyclique saturé à 5 ou 6 chaînons avec un ou deux hétéroatomes choisis indépendamment parmi O, S et N, lequel groupement C₁₋₃alkyle peut être substitué par un ou deux substituants choisis indépendamment parmi oxo, hydroxy, halogéno et C₁₋₄alcoxy et lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
4) C₁₋₅alkylX¹⁰C₁₋₅alkylX¹¹R³² (où X¹⁰ et X¹¹ représentent chacun indépendamment O, S, SO, SO₂, NR³³CO, CONR³⁴, SO₂NR³⁵, NR³⁶SO₂ ou NR³⁷ (où R³³, R³⁴, R³⁵, R³⁶ et R³⁷ représentent chacun indépendamment hydrogène, C₁₋₃alkyle ou C₁₋₃alcoxyC₂₋₃alkyle) et R³² est hydrogène ou C₁₋₃alkyle) ;
5) R³⁸ (où R³⁸ est un groupement hétérocyclique saturé à 5 ou 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy);
6) C₁₋₅alkylR³⁸ (où R³⁸ est tel que défini précédemment);
7) C₂₋₅alcénylR³⁸ (où R³⁸ est tel que défini précédemment) ;
8) C₂₋₅alcynylR³⁸ (où R³⁸ est tel que défini précédemment) ;
9) R³⁹ (où R³⁹ est un groupement pyridone, un groupement phényle ou un groupement hétérocyclique aromatique à 5 ou 6 chaînons avec 1 à 3 hétéroatomes choisis indépendamment parmi O, N et S, lequel groupement pyridone, phényle ou hétérocyclique peut porter jusqu'à 5 substituants choisis indépendamment parmi hydroxy, halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, trifluorométhyle, cyano, CONR⁴⁰R⁴¹ et NR⁴²COR⁴³ (où R⁴⁰, R⁴¹, R⁴² et R⁴³ représentent chacun indépendamment hydrogène, C₁₋₄alkyle ou C₁₋₃alcoxyC₂₋₃alkyle));
10) C₁₋₅alkylR³⁹ (où R³⁹ est tel que défini précédemment);
11) C₂₋₅alcénylR³⁹ (où R³⁹ est tel que défini précédemment);
12) C₂₋₅alcynylR³⁹ (où R³⁹ est tel que défini précédemment) ;
13) C₁₋₅alkylX¹²R³⁹ (où X¹² est O, S, SO, SO₂, NR⁴⁴CO, CONR⁴⁵, SO₂NR⁴⁶, NR⁴⁷SO₂ ou NR⁴⁸ (où R⁴⁴, R⁴⁵, R⁴⁶, R⁴⁷ et R⁴⁸ représentent chacun indépendamment hydrogène, C₁₋₃alkyle ou C₁₋₃alcoxyC₂₋₃alkyle) et R³⁹ est tel que défini précédemment) ;
14) C₂₋₅alcénylX¹³R³⁹ (où X¹³ est 0, S, SO, SO₂, NR⁴⁹CO, CONR⁵⁰, SO₂NR⁵¹, NR⁵²SO₂ ou NR⁵³ (où R⁴⁹, R⁵⁰, R⁵¹, R⁵² et R⁵³ représentent chacun indépendamment hydrogène, C₁₋₃alkyle ou C₁₋₃alcoxyC₂₋₃alkyle) et R³⁹ est tel que défini précédemment) ;
15) C₂₋₅alcynylX¹⁴R³⁹ (où X¹⁴ est 0, S, SO, SO₂, NR⁵⁴CO, CONR⁵⁵, SO₂NR⁵⁶, NR⁵⁷SO₂ ou NR⁵⁸ (où R⁵⁴, R⁵⁵, R⁵⁶, R⁵⁷ et R⁵⁸ représentent chacun indépendamment hydrogène, C₁₋₃alkyle ou C₁₋₃alcoxyC₂₋₃alkyle) et R³⁹ est tel que défini précédemment) ;
16) C₁₋₃alkylX¹⁵C₁₋₃alkylR³⁹ (où X¹⁵ est 0, S, SO, SO₂, NR⁵⁹CO, CONR⁶⁰, SO₂NR⁶¹, NR⁶²SO₂ ou NR⁶³ (où R⁵⁹, R⁶⁰, R⁶¹, R⁶² et R⁶³ représentent chacun indépendamment hydrogène, C₁₋₃alkyle ou C₁₋₃alcoxyC₂₋₃alkyle) et R³⁹ est tel que défini précédemment) ; et
17) C₁₋₃alkylX¹⁵C₁₋₃alkylR³⁸ (où X¹⁵ et R³⁸ sont tels que définis précédemment) ;
n est 0, 1, 2 ou 3 lorsque Z est un cycle hétérocyclique à 6 chaînons et n est 0, 1 ou 2 lorsque Z est un cycle hétérocyclique à 5 chaînons;
m est 0, 1, 2, 3 ou 4 ;
sous forme de base libre ou de sels pharmaceutiquement acceptables de celui-ci, dans la fabrication d'un médicament destiné au traitement et/ou à la prévention de la déficience cognitive légère, de la déficience de la mémoire liée à l'âge, du déclin cognitif lié à l'âge, de la déficience cognitive sans démence, du déclin cognitif léger, du déclin neurocognitif léger, de l'oubli bénin lié à l'âge, de la déficience de la mémoire et de la déficience cognitive.

4. Utilisation selon l'une quelconque des revendications 1 à 3, **caractérisée en ce que** Z est un cycle hétérocyclique à 6 chaînons contenant 1 ou 2 atomes d'azote et R¹ est hydrogène.

5. Utilisation selon l'une quelconque des revendications 1 à 4, **caractérisée en ce que** R² est halogéno, C₁₋₃alkyle, trifluorométhyle, cyano, carbamoyle, *N*-C₁₋₄alkylcarbamoyle, aminosulfonyle ou un groupement R⁴X¹,
où X¹ est CONR⁶R⁷ (où R⁶ est hydrogène ou C₁₋₂alkyle et R⁷ est C₁₋₄alkyle et où R⁴ est lié à R⁷); et
n est 0 ou 1.

6. Utilisation selon l'une quelconque des revendications 1 à 5, **caractérisée en ce que** R³ est R¹⁵X⁷,
où X⁷ est O ; et
R¹⁵ est choisi dans l'un des groupes suivants :
1) hydrogène ou C₁₋₅alkyle ;
3) C₁₋₅alkylX⁹R²⁶ (où X⁹ est O (où R²⁶ est hydrogène ou C₁₋₃alkyle));
4) C₁₋₅alkylX¹⁰C₁₋₅alkylX¹¹R¹² (où X¹⁰ et X¹¹ sont O et R³² est hydrogène ou C₁₋₃alkyle) ;
6) C₁₋₅alkylR³⁸ (où R³⁸ est un groupement hétérocyclique saturé à 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
7) C₂₋₅alcénylR³⁸ (où R³⁸ est tel que défini précédemment) ;
10) C₁₋₅alkylR³⁹ (où R³⁹ est un groupement hétérocyclique aromatique à 5 ou 6 chaînons avec 1 à 3 hétéroatomes choisis indépendamment parmi O, N et S, lequel groupement hétérocyclique peut porter jusqu'à 4 substituants choisis indépendamment parmi hydroxy, halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, trifluorométhyle, cyano, CONR⁴⁰R⁴¹ et NR⁴²COR⁴³ (où R⁴⁰, R⁴¹, R⁴² et R⁴³ représentent chacun indépendamment hydrogène, C₁₋₄alkyle ou C₁₋₃alcoxyC₂₋₃alkyle)) ;
13) C₁₋₅alkylX¹²R³⁹ (où X¹² est O et R³⁹ est tel que défini précédemment) ;
m est 0, 1 ou 2.

7. Utilisation selon l'une quelconque des revendications 1 à 6, **caractérisée en ce que** les composés sont choisis parmi
la 4-(7-azaoxindol-3-yl)-6-méthoxy-7-(2-méthoxyéthoxy)quinazoline,
la 4-(7-azaoxindol-3-yl)-6-méthoxy-7-(3-morpholinopropoxy)quinazoline,
la 4-(7-azaoxindol-3-yl)-7-(2-imidazol-1-yl)éthoxy)-6-méthoxyquinazoline,
la 4-(5,7-diaza-6-méthyloxindol-3-yl)-7-(3-morpholinopropoxy)quinazoline,
la 4-(7-aza-6-chlorooxindol-3-yl)-7-(3-morpholinopropoxy)quinazoline,
la 4-(5,7-diaza-6-méthyloxindol-3-yl)-6-méthoxy-7-(2(1,2,3-triazol-1-yl)éthoxy)quinazoline,
la 4-(5,7-diaza-6-méthyloxind-ol-3-yl)-7-(2-(2-méthoxyéthoxy)éthoxy)quinazoline,
la 4-(7-azaoxindol-3-yl)-7-(3-morpholinopropoxy)quinazoline,
la 4-(7-azaoxindol-3-yl)-7-(2-(2-méthoxyéthoxy)éthoxy)quinazoline,
la 4-(7-azaoxindol-3-yl)-7-(4-morpholinobut-2-én-1-yloxy)quinazoline,
la 4-5,7-diaza-6-méthyloxindol-3-yl)-6-méthoxy-7-(2-(4-pyridyloxy)éthoxy)quinazoline,
la 4-(7-aza-6-chlorooxindol-3-yl)-7-(2-(2-méthoxyéthoxy)éthoxy)quinazoline, et
la 4-(5,7-diaza-6-méthyloxindol-3-yl)-7-(3-(1,1-dioxothiomorpholino)propoxy)quinazoline ;
sous forme d'une base libre ou de sels pharmaceutiquement acceptables de celles-ci.
